# EUROPEAN PATENT APPLICATION

(11) **EP 3 343 224 A1**
(43) Date of publication of application: **04.07.2018**
(21) Application number: 17207284.5
(22) Date of filing: 14.12.2017
(51) Int. Cl.: G01N 33/543

(54) **METHOD AND APPARATUS FOR WASHING, SEPARATION AND MIXING OF IMMUNO MAGNETIC PARTICLES IN AN IMMUNOANALYZER SYSTEM**

(30) Priority: 28.12.2016 JP 2016256215
(71) Applicant: SYSMEX CORPORATION, Kobe-shi Hyogo 651-0073 (JP)
(72) Inventor: KUBO, Takuya, Hyogo, 651-0073 (JP)
(74) Representative: Hoffmann Eitle

(57) **Abstract**

Disclosed is an analysis method including: a component to be analyzed being set; bringing a carrier and a complex that includes the component into contact with each other, to form the complex on the carrier; selecting a washing process in accordance with the component; and analyzing the component, with use of the washing process selected in accordance with the set component.

## Description

### CROSS REFERENCE TO RELATED APPLICATIONS

This application claims priority from prior Japanese Patent Application No. 2016-256215, filed on December 28, 2016, entitled "ANALYSIS METHOD AND ANALYZER", the entire content of which is incorporated herein by reference.

### FIELD OF THE INVENTION

There is an analyzer which analyzes components with use of a process of washing a complex (hereinafter, referred to as washing process for a complex) that includes a component to be analyzed (hereinafter, referred to as target component), the component being contained in a specimen (see Japanese Laid-Open Patent Publication No. 2007-192766, for example).

### BACKGROUND

In the analyzer disclosed in Japanese Laid-Open Patent Publication No. 2007-192766 mentioned above, as shown in FIG. 21, a complex of a target component in a specimen and an antibody is formed on a carrier through antigen-antibody reaction in a reaction unit, and then, in a BF separator, a washing process for the complex is performed by use of a washing liquid. The target component is any of various types of antigens and antibodies, for example. The washing process is a process of separating the complex including the target component, from substances other than the complex by use of a washing liquid, in order to suppress occurrence of a detection signal that is not derived from the target component. In laboratory tests, large quantities of specimens are handled, and thus, the washing process is often used in common. In the analyzer disclosed in Japanese Laid-Open Patent Publication No. 2007-192766 mentioned above, a common and single kind of washing process is performed on the complex irrespective of the kind of the target component.

### SUMMARY OF THE INVENTION

In recent years, analyzers are desired to be able to measure a wider variety of target components, than conventional analyzers. However, vigorous study by the inventors of the present application has revealed that: in a case where a common washing process is performed in measurement of a plurality of target components, measurement is hindered by influence of the washing process with respect to some specific target components; and measurement at a sufficient accuracy becomes difficult to be performed.

The present invention is directed to suppressing hindrance of measurement due to influence of a washing process for a complex even when measurement of a plurality of target components is performed.

In order to solve the above problem, the inventors of the present application conducted vigorous study and found that: structural or functional change in the complex could be caused depending on the details of a washing process; and thus, by selecting an appropriate washing process in accordance with the target component, it becomes possible to perform the measurement at sufficient accuracy without the measurement of the target component being hindered. That is, an analysis method according to a first aspect of this invention includes: a component (80) to be analyzed being set; bringing a carrier (82) and a complex (81) that includes the component (80) into contact with each other, to form the complex (81) on the carrier (82); selecting a washing process in accordance with the component (80); and analyzing the component (80), with use of the washing process selected in accordance with the set component (80). Here, "washing process" means a process of removing components unnecessary for measurement and present in the measurement sample other than the carrier (82) that includes the complex (81). The components unnecessary for the measurement include: the target component (80) and reagent components that have been non-specifically adsorbed to the complex (81), the carrier (82), the surface of the reaction container, or the like, without forming the complex (81); other substances such as contaminant substances having entered from outside; and the like.

In the analysis method according to the first aspect, as described above, a washing process for the complex (81) is selected in accordance with the component (80) to be analyzed, and the component (80) is analyzed with use of the washing process selected in accordance with the set component (80). Accordingly, for each target component (80), the washing process for the complex (81) can be performed by use of a washing process suitable for measurement of the target component (80). That is, in a case where measurement of a plurality of target components (80) is performed, even with respect to a specific target component (80) for which measurement is hindered by a general washing process, the measurement of the target component (80) can be performed with use of a washing process of which details, such as the kind of the washing liquid (70) to be used, the number of times of washing, and the washing time period, are appropriately selected. Thus, even in a case where measurement of a plurality of target components (80) is performed by an analyzer (100), the measurement can be suppressed from being hindered by influence of the washing process for the complex (81). In addition, the above finding by the inventors of the present application suggests that, also with respect to a target component (80) for which a common washing process is conventionally performed, a more suitable washing process can be selected. That is, also with respect to a general target component (80) for which a common washing process is conventionally performed, the measurement accuracy can be further improved by selecting an appropriate washing process.

Preferably, in the analysis method according to the first aspect described above, when lipoprotein is set as the component (80) to be analyzed, the analysis method includes forming a complex (81c) that includes: lipoprotein (80B) having tagged cholesterol (86a) incorporated therein; and an antibody (86b) that binds to the lipoprotein. It should be noted that "lipoprotein" is a substance in which lipid and apoprotein are bound to each other. "Apoprotein" has a structure in which a par of protein is removed. Here, since lipoprotein contains lipid, there are cases where lipoprotein undergoes structural change during a washing process. Thus, according to the present invention, even in a case where lipoprotein is included in the complex (81), an appropriate washing process can be selected. Thus, the measurement can be effectively suppressed from being hindered by influence of the washing process.

In the analysis method according to the first aspect described above, preferably, a washing liquid (70) to be used is selected from a plurality of kinds of washing liquids (70) in which at least washing liquid components contained therein or proportions of the washing liquid components are different. With this configuration, for each target component (80), it is possible to select a washing liquid (70) that contains a washing liquid component particularly suitable for the measurement, or that contains a washing liquid component in a proportion suitable for the measurement, to perform the washing process. As a result, the measurement can be effectively suppressed from being hindered by influence of the washing process.

In this case, preferably, a washing liquid (70) that does not contain any surfactant or that contains a surfactant at a concentration of less than 1.1 g/L is selected in accordance with the component (80) to be analyzed. With this configuration, with resect to the target component (80) that is subjected to influence by the surfactant, influence of the surfactant contained in the washing liquid (70) can be suppressed. For example, in the case of a target component (80) containing lipid, structural change could be caused by influence such as formation of micelle by the surfactant. Thus, by selecting a washing liquid (70) having a surfactant concentration of less than 1.1 g/L, it is possible to effectively suppress the influence of the washing liquid (70). Micelle is a structure composed of a collection of particles of one of liquids that do not mix each other, the particles being surrounded by a surfactant.

In the above-described configuration in which a washing liquid (70) is selected from a plurality of kinds of washing liquids (70) in which washing liquid components contained therein or proportions of the washing liquid components are different, preferably, a washing liquid (70) that contains a predetermined washing liquid component is selected in accordance with the component (80) to be analyzed. With this configuration, for each target component (80), it is possible to select a washing liquid (70) that contains a washing liquid component particularly suitable for the measurement. Thus, by selecting a washing liquid (70) that contains a washing liquid component having a particularly high washing effect, it is possible to improve the measurement accuracy, and to reduce the time period necessary for the washing process, the amount of the washing liquid (70) to be used, the number of times of washing, and the like, for example.

In this case, preferably, the predetermined washing liquid component is a salt. "Salt" is a compound in which an anion derived from an acid and a cation derived from a base are bound to each other through an ionic bond. With this configuration, by using a washing liquid (70) containing a salt at an appropriate concentration, it is possible to enhance the washing effect of the washing liquid (70). As a result, the background signal can be effectively reduced, and the measurement accuracy (signal/background ratio) can be further improved.

In the above-described configuration in which a washing liquid (70) is selected from a plurality of kinds of washing liquids (70) in which washing liquid components contained therein or proportions of the washing liquid components are different, preferably, the washing liquid is a washing liquid (75) for a liquid distribution channel. With this configuration, the washing liquid (75) for washing the liquid distribution channel can be used as a washing liquid (70) to be used in washing the complex (81). That is, different from the case where a plurality of kinds of washing liquids (70) for the washing process for the complex (81) are prepared, increase in the number of kinds of washing liquids stored for analysis can be suppressed by using the washing liquid (75) for washing the liquid distribution channel.

In the above-described configuration in which a washing liquid (70) is selected from a plurality of kinds of washing liquids (70) in which washing liquid components contained therein or proportions of the washing liquid components are different, preferably, the analysis method includes preparing a washing liquid (70) to be used in the washing process, by mixing one washing liquid and another washing liquid from among a plurality of washing liquids (71a to 71c). With this configuration, from among the plurality of kinds of washing liquids (71a to 71c) prepared in advance, a washing liquid (70) having a more diverse composition can be prepared. Thus, the kinds of selectable washing liquids (70) can be easily increased.

In the analysis method according to the first aspect described above, preferably, the component (80) to be analyzed being set includes: obtaining information that specifies the component (80); and selecting the washing process on the basis of the obtained information that specifies the component (80). With this configuration, by obtaining the information that specifies the target component (80), it is possible to easily select an appropriate washing process in accordance with the target component (80), to perform the analysis.

In this case, preferably, the information that specifies the target component (80) is a measurement order (91) in which a measurement item is set for each specimen, and the analysis method includes obtaining the measurement order (91) for the specimen, and selecting the washing process in accordance with the measurement item in the measurement order (91). With this configuration, by use of the measurement order (91) obtained for performing analysis of the target component (80), selection of the washing process can also be performed. Thus, there is no need to separately provide the analyzer (100) with information for selecting a washing process, and an appropriate washing process in accordance with the target component (80) can be easily selected.

In the analysis method according to the first aspect described above, preferably, the washing process includes: magnetically collecting the complex (81); supplying a washing liquid (70) to be used in the washing process; and removing the washing liquid (70). With this configuration, through the supply and removal of the washing liquid (70), components unnecessary for the measurement other than the carrier (82) that includes the complex (81) can be effectively removed.

In the analysis method according to the first aspect described above, preferably, analysis of the component (80) is performed through immunoassay using antigen-antibody reaction. In the immunoassay using antigen-antibody reaction, for the purpose of improving the detection accuracy, it is desired to perform an effective washing process in order to suppress non-specific adsorption of the target component (80), reagent components, and other substances such as contaminant substances from outside, while maintaining the structure and function of the complex (81). Thus, the analysis method according to the present invention is effective in particular.

In the analysis method according to the first aspect described above, preferably, the carrier (82) is a solid-phase particle. With this configuration, the complex (81) can be formed on the solid-phase particle in the reaction container (90). Thus, for example, compared with a case where a plate having the carrier (82) immobilized thereon or the like is used, handling of the sample and the reaction container (90) in the analyzer (100) can be facilitated.

In this case, preferably, the solid-phase particle is a magnetic particle (82a). With this configuration, the magnetic particles (82a) in the reaction container (90) can be captured by magnetic force. As a result, when the complex (81) is washed in the washing process, the magnetic particles (82a) are easily captured by magnetic force, whereby the liquid component other than the magnetic particles (82a) can be easily removed.

The analysis method according to the first aspect described above, preferably includes: performing a first washing process by use of the selected washing process; causing the complex (81) and a labeled substance (83) to be bound to each other, after the first washing process; performing a second washing process by use of the selected washing process; and measuring a signal based on the labeled substance (83), after the second washing process. With this configuration, a higher washing effect can be obtained by performing the first washing process and the second washing process. Also in this case, details of an appropriate washing process can be selected, and thus, the measurement can be effectively suppressed from being hindered by influence of the washing process.

An analyzer (100) according to a second aspect of this invention includes: a setting unit (14) configured to set a component (80) to be analyzed; a reaction unit (10) configured to bring a carrier (82) and a complex (81) that includes the component (80) into contact with each other, to form the complex (81) on the carrier (82); a washing unit (11) configured to perform a washing process selected in accordance with the set component (80), the washing process being for washing the complex (81) formed in the reaction unit (10); an analysis unit (13) configured to analyze the component (80) after the washing process has been performed by the washing unit (11); and a controller (12) configured to select the washing process in accordance with the component (80).

As described above, the analyzer (100) according to the second aspect includes: the washing unit (11) configured to perform a washing process selected in accordance with the set component (80); and the controller (12) configured to select the washing process in accordance with the component (80). Accordingly, for each target component (80), a washing process suitable for the measurement of the target component (80) can be selected, to perform the washing process for the complex (81). That is, in a case where measurement of a plurality of target components (80) is performed, even with respect to a specific target component (80) for which the measurement is hindered by a general washing process, the measurement of the target component (80) can be performed, with use of a washing process of which details, such as the kind of the washing liquids (70) to be used, the number of times of washing, and the washing time period, are appropriately selected. Thus, even in a case where measurement of a plurality of target components (80) is performed by the analyzer (100), the measurement can be suppressed from being hindered by influence of the washing process for the complex (81). Also with respect to a target component (80) for which a common washing process is conventionally performed, the measurement accuracy can be further improved by selecting an appropriate washing process.

In the analyzer (100) according to the second aspect described above, preferably, the controller (12) selects a washing liquid (70) to be used in the washing process for the complex (81), in accordance with the target component (80). With this configuration, for each target component (80), a washing liquid (70) having a composition particularly suitable for the measurement can be selected, to perform the washing process. As a result, the measurement can be effectively suppressed from being hindered by influence of the washing process.

The analyzer (100) according to the second aspect described above preferably includes a washing liquid storage part (23) configured to store a plurality of washing liquids (70), wherein the controller (12) selects the washing liquid (70) to be used in the washing process from among the washing liquids (70) stored in the washing liquid storage part (23). With this configuration, for example, different from a case where the washing liquid (70) is supplied from outside the apparatus, the analyzer (100) can singly select a washing liquid (70) and perform the washing process. As a result, the apparatus configuration can be simplified, and analysis of the target component (80) can be efficiently performed.

In this case, preferably, the washing liquid storage part (23) includes a first storage part (23a) configured to store a washing liquid (70) to be used in the washing process, and the controller (12) selects the washing liquid (70) stored in the first storage part (23a), as the washing liquid (70) to be used in the washing process. With this configuration, from among the washing liquids (70) for the washing process stored in the first storage part (23a), an appropriate washing liquid (70) can be easily selected to be used in the washing process.

In the above-described configuration provided with the washing liquid storage part (23), preferably, the washing liquid storage part (23) includes a second storage part (23b) configured to store a washing liquid (75) for washing a liquid distribution channel, and the controller (12) selects the washing liquid (75) stored in the second storage part (23b), as the washing liquid (70) to be used in the washing process. With this configuration, the washing liquid (75) for washing the liquid distribution channel can be used in washing the complex (81). That is, different from a case where a plurality of kinds of the washing liquids (70) for the washing process for the complex (81) are prepared, increase in the number of kinds of the washing liquids (70) stored in the analyzer (100) can be suppressed by using the washing liquid (75) for washing the liquid distribution channel.

In the above-described configuration provided with the washing liquid storage part (23), preferably, the analyzer includes: a reagent dispenser (32a to 32c) configured to dispense a reagent into a reaction container (90); and a reagent storage part (35) configured to store a reagent to be used in analysis of the target component (80), wherein the controller (12) selects a washing liquid (70) to be used in the washing process from the washing liquid (70) stored in the reagent storage part (35). With this configuration, the washing liquid (70) can be stored by use of the reagent storage part (35) for storing a reagent. As a result, when compared with a case where a storage part for storing a plurality of kinds of washing liquids (70) is separately provided, without making the apparatus configuration significantly different from conventional apparatus configurations, a large number of kinds of washing liquids (70) can be easily stored and a washing liquid (70) can be selected in accordance with the target component (80).

In the above-described configuration provided with the washing liquid storage part (23), preferably, the analyzer includes: a supply channel (56) configured to supply a washing liquid (70) from the washing liquid storage part (23) to the washing unit (11), wherein when switching one washing liquid (70) to another washing liquid (70), the controller (12) performs control of washing, with the another washing liquid (70), the supply channel (56) through which the one washing liquid (70) has been sent. With this configuration, in a case where the washing liquids (70) are supplied through a common supply channel (56), one previously-used washing liquid (70) can be prevented from being mixed with another subsequently-used washing liquid (70), and can be prevented from being supplied to the washing unit (11) in a state of being mixed with the another subsequently-used washing liquid (70).

In the above-described configuration provided with the washing liquid storage part (23), preferably, the analyzer includes: a plurality of supply channels (56) each configured to supply a washing liquid (70) from the washing liquid storage part (23) to the washing unit (11), wherein the controller (12) performs control of selecting a separate supply channel (56) in accordance with the selected washing liquid (70), to send the selected washing liquid (70). With this configuration, the supply channels (56) for a plurality of kinds of washing liquids (70) can be individually provided. As a result, when washing liquids (70) are supplied to the washing unit (11), different kinds of washing liquids (70) can be prevented from being mixed.

In the analyzer (100) according to the second aspect described above, preferably, the washing unit (11) includes a plurality of washing ports (44) each configured to perform the washing process for the complex (81), and the controller (12) performs control of selecting a washing liquid (70) to be supplied, for each of the washing ports (44). With this configuration, without increasing the number of the washing ports (44), various types of washing liquids (70) can be selected and supplied.

The analyzer (100) according to the second aspect described above preferably includes an input unit (55) configured to receive an operation input performed by a user, wherein the controller (12) selects a washing liquid (70) to be used in the washing process on the basis of information received from the input unit (55). With this configuration, through the input unit (55), the user is allowed to determine the washing liquid (70) to be used, or the controller (12) can perform selection such that the controller (12) determines a washing liquid (70) to be used on the basis of the information inputted by the user, and then, the determined washing liquid (70) is supplied to the washing unit (11). As a result, an appropriate washing liquid (70) can be easily selected.

Thus, also in a case where measurement of a plurality of target components is performed by the analyzer, the measurement can be suppressed from being hindered by influence of the washing process for the complex.

### BRIEF DESCRIPTION OF THE DRAWINGS

FIG. 1 is a diagram for describing the overview of an analyzer and an analysis method according to one embodiment;
FIG. 2A is a plan view showing a first stage in one configuration example of the analyzer;
FIG.2B is a plan view showing a second stage in one configuration example of the analyzer;
FIG. 3 is a schematic side view showing a configuration example of a washing unit;
FIG. 4 is a schematic diagram showing a method for washing a complex performed by the washing unit;
FIG. 5 is a diagram for describing a first selection method for selecting a washing liquid;
FIG. 6 is a diagram for describing a second selection method for selecting a washing liquid;
FIG. 7 is a diagram for describing a third selection method for selecting a washing liquid;
FIG. 8 is a diagram showing a configuration example of preparing a washing liquid;
FIG. 9 is a diagram for describing a first example of a washing liquid supplying method in the washing unit;
FIG. 10 is a diagram for describing a second example of the washing liquid supplying method in the washing unit;
FIG. 11 is a diagram for describing a third example of the washing liquid supplying method in the washing unit;
FIG. 12 is a flow chart for describing a control process of selecting a washing liquid;
FIG. 13 is a diagram for describing one example of a method for specifying a target component;
FIG. 14 is a diagram for describing a configuration example in which measurement operation is suspended and a washing liquid selection input is received;
FIG. 15 is a diagram for describing a configuration example in which measurement operation is skipped and the washing process is collectively performed for each kind of washing liquid;
FIG. 16A is a diagram showing a measurement method for which the target component is hepatitis B surface antigen;
FIG. 16B is a diagram showing a measurement method for which the target component is high density lipoprotein;
FIG. 17 is a flow chart for describing measurement process operation performed by the analyzer;
FIG. 18 is a diagram showing the compositions of washing liquids A to D used in Example 1;
FIG. 19 is a diagram showing a detection result of the target component for each washing liquid in Example 1;
FIG. 20 is a diagram showing a detection result of the target component using the washing liquid C in Example 2; and

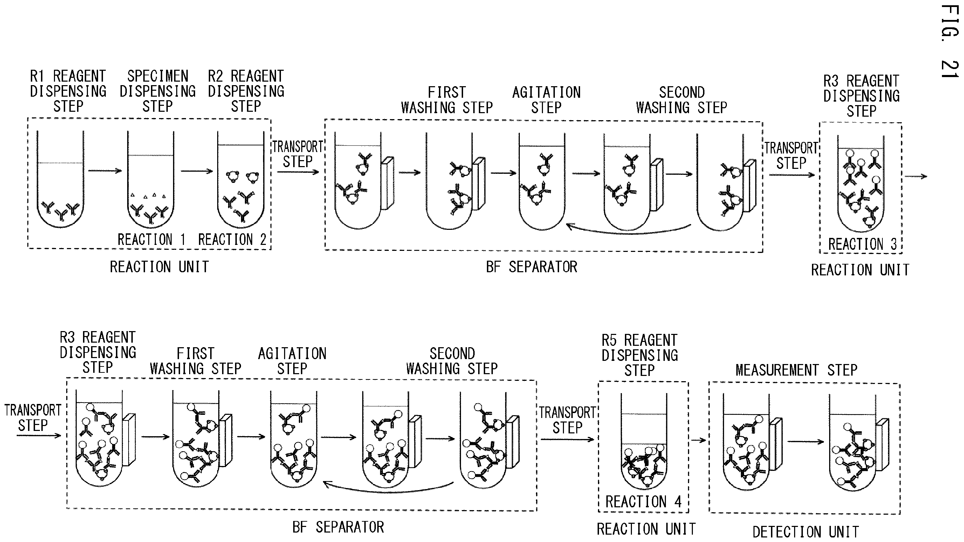

FIG. 21 is a diagram showing the flow of a process performed in a conventional analyzer.

### DETAILED DESCRIPTION OF THE PREFERRED EMBODIMENTS

Hereinafter, embodiments are described with reference to the drawings.

### [Overview of analyzer and analysis method]

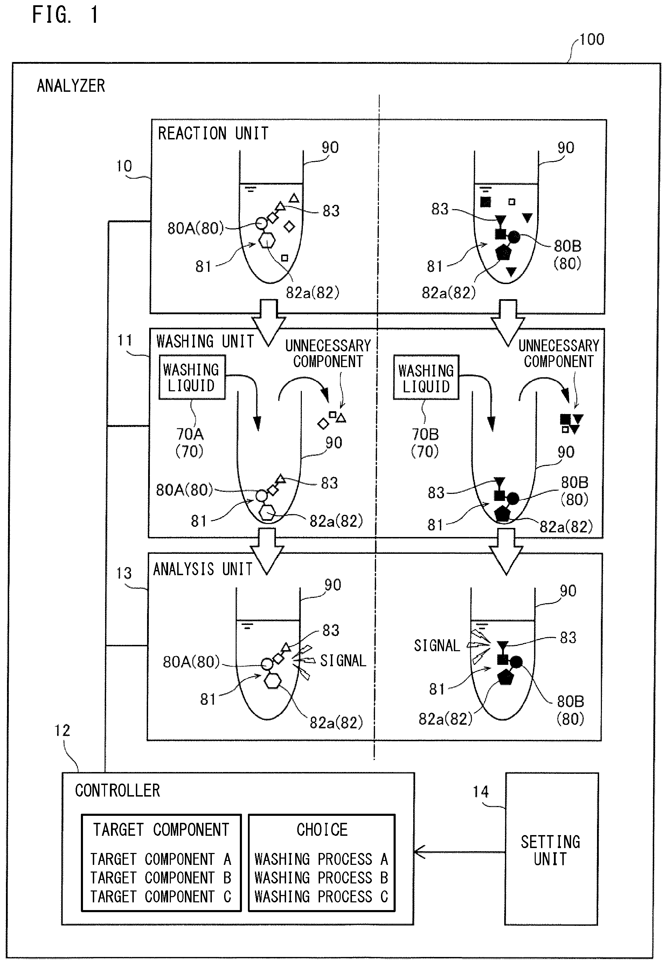

First, the overview of an analyzer 100 according to one embodiment is described with reference to FIG. 1.

The analyzer 100 is an analyzer which analyzes a component to be analyzed 80 (hereinafter, referred to as target component 80). The analyzer 100 of the present embodiment analyzes the target component 80, with use of a washing process for a complex 81 including the target component 80 contained in a specimen. That is, the analyzer 100 performs measurement of the target component 80 by use of a heterogeneous assay which is a method in which: a complex 81 is formed by causing a target component 80 and a substance that specifically reacts with the target component 80 to bind to each other; and the formed complex 81 is separated (BF separation) through a washing process from free substances not having been involved in the reaction; and then, the complex 81 is measured.

The target component 80 and the complex 81 to be formed are not limited in particular. The target component 80 is a substance contained in a biological specimen such as blood, urine, or tissue. The complex 81 includes: the target component 80; and a labeled substance 83 that specifically reacts with the target component 80, for example. Between the target component 80 and the labeled substance 83, a capture substance for binding these may be mediated. By detecting a signal based on the labeled substance 83 in the complex 81, it becomes possible to measure the target component 80.

The analyzer 100 can measure various target components 80 by varying the combination of the target component 80 and the substance that specifically reacts with the target component 80.

When a free substance not having been involved in reaction or a contaminant substance having entered from outside during measurement are present in a measurement sample, a non-specific detection signal not derived from the target component 80 is generated, and this causes a reduced signal-noise ratio. The washing process is performed in order to remove, from the measurement sample, unnecessary components which become a generation source of the non-specific detection signal not derived from the target component 80. The unnecessary components include: components that have been non-specifically adsorbed to the complex 81, a carrier 82, the surface of a reaction container 90, or the like, without forming the complex 81; other substances such as contaminant substances having entered from outside; and the like.

The analyzer 100 includes a reaction unit 10, a washing unit 11, a controller 12, an analysis unit 13, and a setting unit 14. In the example shown in FIG. 1, the analyzer 100 performs measurement operation by use of the reaction container 90 which accommodates the target component 80. Instead of using the reaction container 90, the analyzer 100 may perform measurement operation, with the target component 80 accommodated in an accommodation part provided in the analyzer 100.

The controller 12 is implemented as a computer mainly composed of: a processor such as a CPU (central processing unit) or an FPGA (field-programmable gate array); a storage unit implemented as a volatile memory, a nonvolatile memory, or a combination thereof, for example. The controller 12 performs operation control of the analyzer 100 by executing a program stored in the storage unit.

The setting unit 14 has a function of setting a target component 80. The setting unit 14 receives from a user through, for example, an input device, an input of information specifying a target component 80 contained in a specimen. The setting unit 14 sets the specified target component 80 as a measurement item. In a case where the information specifying the target component 80 is managed in the form of a measurement order for each specimen, the setting unit 14 may specify the target component 80 by causing a reader device, for example, to read specimen identification information attached to a specimen container, and by obtaining the measurement order that corresponds to the specimen identification information. The setting unit 14 can be implemented by a computer similar to the controller 12, or may be implemented by a control device that is used in common with the controller 12.

The reaction container 90 is a disposable container made of resin, for example. The reaction container 90 has a substantially cylindrical shape having a closed bottom, for example, and can hold various types of liquids, such as a specimen, poured through the upper opening thereof.

The reaction unit 10 has a function of forming on the carrier 82 the complex 81 including the target component 80, by bringing the carrier 82 and the target component 80 contained in the specimen into contact with each other in the reaction container 90. For example, the reaction unit 10 facilitates the reaction for forming the complex 81 by heating the reaction container 90. In this case, the reaction unit 10 holds the reaction container 90 that accommodates: the specimen containing the target component 80; and a reagent containing the carrier 82, and maintains the reaction container 90 at a predetermined reaction temperature for a predetermined time period.

The carrier 82 is not limited in particular as long as the carrier 82 can carry the complex 81 by being bound to the complex 81. The carrier 82 may be: a plate having immobilized thereon a binding substance that binds to the complex 81; or a known particle, for example. Preferably, the carrier 82 is a solid particle such as a magnetic particle, a latex particle, a gelatin particle, for example. In this case, the complex 81 can be formed on the solid particle in the reaction container 90. Thus, for example, compared with a case where a plate or the like having the carrier 82 immobilized thereon is used, handling of the sample and the reaction container 90 in the analyzer 100 can be facilitated.

More preferably, the solid particle is a magnetic particle 82a. In this case, the magnetic particles 82a in the reaction container 90 can be captured by magnetic force. As a result, when the complex 81 is washed in the washing process, the magnetic particles 82a are captured by magnetic force, whereby the liquid component other than the magnetic particles 82a can be easily removed.

The magnetic particles may be any particle that contains a magnetic material as the base material thereof. For example, magnetic particles that use, as the base material, Fe₂O₃ and/or Fe₃O₄, cobalt, nickel, ferrite, magnetite, or the like can be used. The magnetic particle 82a may be coated with a binding substance for binding to the target component 80, or may be bound to the target component 80 through a capture substance for causing the magnetic particle 82a and the target component 80 to be bound to each other.

In the reaction unit 10, the carrier 82, the target component 80, and the labeled substance 83 are directly or indirectly bound to one another in the reaction container 90, whereby the complex 81 is formed on the carrier 82.

The washing unit 11 has a function of performing a washing process for the complex 81 formed in the reaction unit 10, the washing process having been selected in accordance with a target component 80 that has been set. The washing process is a process of removing unnecessary components present in the measurement sample other than the carrier 82 that includes the complex 81. For example, the washing unit 11 collects the carrier 82 carrying the complex 81 to a predetermined position in the reaction container 90, and removes the liquid other than the carrier 82. For collecting the carrier 82, a method such as centrifugation, capture by an electric field using the principle of electrophoresis, or magnetic collection using magnetic force may be employed.

In the example shown in FIG. 1, after removing the liquid, the washing unit 11 supplies a washing liquid 70 into the reaction container 90 and washes off unnecessary components non-specifically adsorbed to the inner surface of the reaction container 90 and to the carrier 82. In order to efficiently remove the unnecessary components, the washing unit 11 repeats supply of the washing liquid 70 and removal of the liquid a plurality of times, for example.

The analysis unit 13 has a function of analyzing the target component 80 after the washing process has been performed by the washing unit 11. The analysis unit 13 includes a detector which detects a signal based on the labeled substance 83 in the complex 81, for example.

The labeled substance 83 contains a label that specifically binds to the target component 80 and that is measurable by the analysis unit 13. In a case where a capture substance is used, the labeled substance 83 may bind to the capture substance, to be bound to the target component 80 through the capture substance. Examples of the label contained in the labeled substance 83 include enzyme, fluorescent substance, and radioisotope. Examples of the enzyme include alkaline phosphatase (ALP), peroxidase, glucose oxidase, tyrosinase, and acid phosphatase. As the fluorescent substance, fluorescein isothiocyanate (FITC), green fluorescent protein (GFP), luciferin, boron dipyrromethene (BODIPY (trade mark)), or the like can be used. As the radioisotope, 1251, 14C, 32P, 3H, or the like can be used.

When the label is an enzyme, a substrate for the enzyme of the labeled substance 83 may be appropriately selected from well-known substrates in accordance with the enzyme. When the enzyme is alkaline phosphatase, examples of the substrate include: chemiluminescent substrates such as CDP-Star (registered-trademark), (disodium 4-chloro-3-(methoxyspiro[1,2-dioxetane-3,2'-(5'-chloro)tricyclo[3.3.1.13,7]decan]-4-yl)phenylphosphate), and CSPD (registered-trademark) (disodium 3-(4-methoxyspiro[1,2-dioxetane-3,2-(5-chloro)tricyclo[3.3.1.13,7]decane]-4-yl)phenylphosphate); luminescent substrates such as p-nitrophenyl phosphate, 5-bromo-4-chloro-3-indolyl phosphate (BCIP), 4-nitro blue tetrazolium chloride (NBT), and iodonitrotetrazolium (INT); fluorescent substrates such as 4-methylumbelliferyl phosphate (4MUP); chromogenic substrates such as 5-bromo-4-chloro-3-indolyl phosphate (BCIP), disodium 5-bromo-6-chloro-indolyl phosphate, and p-nitrophenyl phosphate; and the like.

The analysis unit 13 performs analysis of the target component 80, by a detector detecting a signal based on the labeled substance 83 contained in the complex 81 accommodated in the reaction container 90. The analysis unit 13 detects a signal from a measurement sample in the reaction container 90 having been subjected to a washing process performed by the washing unit 11. The detection may be performed by an appropriate method according to the kind of the label used for the labeled substance 83, and the measurement method is not limited in particular. For example, in a case where the labeled substance 83 is an enzyme, the detection can be performed by measuring the light, color, or the like that is generated as a result of causing a substrate to react with the enzyme. As the detector in this case, a spectrophotometer, a luminometer, or the like can be used. In a case where the labeled substance 83 is a radioisotope, a scintillation counter or the like can be used as the detector.

Here, when the analyzer 100 performs measurement of various target components 80, there are cases in which, with respect to a specific target component 80, measurement is hindered by influence of the washing process, and measurement at a sufficient accuracy becomes difficult to be performed. For example, there are cases where a washing liquid component contained in the washing liquid 70 acts on the complex 81 to cause structural or functional change in the complex 81. In such a case, the target component 80 or the labeled substance 83 could be separated from the complex 81 or deteriorated, and thus, the measurement could be hindered.

Thus, in the present embodiment, the controller 12 is configured to perform control of selecting a washing process in accordance with the target component 80 that has been set. As a result of the controller 12 selecting the washing process, the washing unit 11 performs the washing process selected in accordance with the target component 80. The washing processes as choices are, for example, different in the kind of the washing liquid 70 to be used in the washing process, or different in part or all of the washing method. The washing method may include a supply amount of the washing liquid 70, the number of times of replacement of the washing liquid 70, a washing time period, and the like.

For example, with reference to FIG. 1, a state in which measurement of a certain kind of target component 80A is performed is shown on the left side in FIG. 1, and a state in which measurement of another kind of target component 80B is performed is shown on the right side in FIG. 1. If measurement of the target component 80A is set by the setting unit 14, a washing process A is selected which uses a washing liquid 70A of a kind that corresponds to the target component 80A. If measurement of the target component 80B is set by the setting unit 14, a washing process B is selected which uses another washing liquid 70B of a kind that corresponds to the target component 80B. It should be noted that the kind of the washing liquid 70 is a classification in which the composition of components of the washing liquid 70 is common. The concept of "composition" includes components contained in the washing liquid and the content proportion or the concentration of the components.

As described above, the analyzer 100 of the present embodiment includes: the setting unit 14 configured to set a target component 80; the reaction unit 10 configured to bring the carrier 82 and the complex 81 that includes the target component 80 into contact with each other, to form the complex 81 on the carrier 82; the washing unit 11 configured to perform a washing process selected in accordance with the set target component 80, the washing process being for washing the complex 81 formed in the reaction unit 10; the analysis unit 13 configured to analyze the target component 80 after the washing process has been performed by the washing unit 11; and the controller 12 configured to select the washing process in accordance with the target component 80. Accordingly, for each target component 80, a washing process suitable for the measurement of the target component 80 can be selected, to perform the washing process for the complex 81. That is, in a case where measurement of a plurality of target components 80 is performed, even with respect to a specific target component 80 for which the measurement is hindered by a general washing process, the measurement of the target component 80 can be performed, with use of a washing process of which details, such as the kind of the washing liquid 70 to be used, the number of times of washing, and the washing time period, are appropriately selected. Thus, even in a case where measurement of a plurality of target components 80 is performed by the analyzer 100, the measurement can be suppressed from being hindered by influence of the washing process for the complex 81. Also with respect to a target component 80 for which a common washing process is conventionally performed, the measurement accuracy can be further improved by selecting an appropriate washing process.

In the present embodiment, through operation of the analyzer 100, an analysis method is performed which executes: a step in which a target component 80 is set; a step of bringing the carrier 82 and the complex 81 that includes the target component 80 into contact with each other, to form the complex 81 on the carrier 82; a step of selecting a washing process in accordance with the target component 80; a step of analyzing the target component 80 with use of the washing process selected in accordance with the set target component 80. Accordingly, for each target component 80, the washing process for the complex 81 can be performed by use of a washing process suitable for the measurement of the target component 80. That is, in a case where measurement of a plurality of target components 80 is performed, even with respect to a specific target component 80 for which the measurement is hindered by a general washing process, the measurement of the target component 80 can be performed with use of a washing process of which details, such as the kind of the washing liquid 70 to be used, the number of times of washing, and the washing time period, are appropriately selected. Thus, even in a case where measurement of a plurality of target components 80 is performed by the analyzer 100, the measurement can be suppressed from being hindered by influence of the washing process for the complex 81.

It should be noted that, measurement could be hindered by influence of a component of the washing liquid 70, whereas there could be a component that is particularly suitable for washing a specific target component 80. In such a case, a washing process suitable for washing the specific target component 80 may be selected. In this case, also with respect to a general target component 80 for which a common washing process is conventionally performed, unnecessary components can be efficiently removed by selecting an appropriate washing process and performing the selected washing process. As a result, the measurement accuracy can be further improved.

Even in a case where the target components 80 are the same, the substance constituting the complex 81 to be formed, the carrier 82, and the like could be different depending on the measurement item. In such a case, the washing process for the complex 81 is selected in accordance with the measurement item to be measured by the analyzer 100, in addition to the target component 80. Accordingly, in a case where a plurality of measurement items are present for the same target component 80, and reagents to be used for the respective measurement items are different, more appropriate washing processes suitable for the respective measurement items can be selected.

Determination of the washing process to be used in washing of the complex 81 may be performed by the controller 12 or by the user in the form of the controller 12 receiving an operation input of the user. The controller 12 selects the determined washing process, and controls the analyzer 100 such that the washing unit 11 performs the selected washing process.

Preferably, the controller 12 selects the washing liquid 70 to be used in the washing process for the complex 81, in accordance with the target component 80. Accordingly, for each target component 80, a washing liquid 70 having a composition particularly suitable for the measurement can be selected, to perform the washing process. As a result, the measurement can be effectively suppressed from being hindered by influence of the washing process.

The washing liquid 70 is selected from among a plurality of choices specified in advance, for example. The washing liquids 70 as the choices have different compositions with one another. That is, a washing liquid 70 to be used is selected from among a plurality of kinds of washing liquids 70 in which at least the washing liquid components contained therein or the proportions of the washing liquid components are different. Accordingly, for each target component 80, a washing liquid 70 having a component and a component proportion particularly suitable for the measurement can be selected, to perform the washing process.

Each washing liquid 70 to be used in the analyzer 100 that handles biological specimens is configured to include a buffer, a surfactant, a salt, etc., for example. Each washing liquid 70 as a choice can include a component other than a component to be used as a washing liquid in general. Each washing liquid 70 as a choice may have a composition usable in common among a plurality of target components 80, or may have a specific composition suitable only for a specific target component 80.

On the basis of a selection criterion for the washing liquid 70, a washing liquid 70 that does not include a predetermined washing liquid component or that has a predetermined washing liquid component having a predetermined concentration or lower is selected in accordance with the target component 80, for example. Accordingly, for each target component 80, if there is a component inappropriate for the measurement, a washing liquid 70 can be selected that does not contain the component inappropriate for the measurement or that contains the inappropriate component only at such a concentration that would not influence the measurement.

Specifically, in accordance with the target component 80, a washing liquid 70 is selected that does not contain any surfactant or that contains a surfactant at a concentration of less than 1.1 g/L, for example. Accordingly, for the target component 80 that would be influenced by the surfactant, the influence of the surfactant contained in the washing liquid 70 can be suppressed. A surfactant is often contained in a washing liquid in general. However, a system in which a hydrophilic substance and a hydrophobic substance are present in the reaction container 90 could be influenced by the surfactant. For example, in the case of a target component 80 containing lipid, structural change could be caused by influence of micelle formed by the surfactant, for example. Thus, the influence of the washing liquid 70 can be effectively suppressed by selecting a washing liquid 70 having a surfactant concentration of less than 1.1 g/L.

As the kind of the surfactant, there are nonionic surfactant, amphoteric surfactant, anionic surfactant, cationic surfactant, and the like. If the influence on the measurement differs depending on the kind of the surfactant, choices of washing liquids having different kinds of surfactants may be provided.

On the basis of a selection criterion for the washing liquid 70, a washing liquid 70 that contains a predetermined washing liquid component is selected in accordance with the target component 80, for example. Accordingly, if there is a washing liquid component that does not have influence on the measurement for each target component 80 and that can enhance the washing effect, such a washing liquid 70 that contains the washing liquid component particularly suitable for the measurement can be selected. Thus, by selecting a washing liquid 70 containing a washing liquid component having a particularly high washing effect, it is possible to improve the measurement accuracy, and to reduce the time period necessary for the washing process, the amount of the washing liquid 70 to be used, the number of times of washing, and the like.

Specifically, the predetermined washing liquid component is a salt, for example. "Salt" is a compound in which an anion derived from an acid and a cation derived from a base are bound to each other through an ionic bond. The washing effect of the washing liquid 70 can be enhanced by using a washing liquid 70 containing a salt having an appropriate concentration. As a result, the background signal can be effectively reduced, whereby the measurement accuracy can be further improved. The kind of salt is not limited in particular. The salt is composed of ions present in vivo, and is NaCl, for example. The salt may be KCl, Na₂HPO₄, or KH₂PO₄, for example.

Regarding a combination of a target component 80 and a corresponding washing liquid 70, for example, so as to correspond to a target component 80 that contains lipid, a washing liquid 70 in which the surfactant content is zero or reduced to a low concentration is selected. For example, in a case where the target component 80 is an enzyme and the enzyme is captured to measure the function, a washing liquid 70 that does not contain metal that hinders the enzymatic reaction is selected.

For example, in a case where the target component 80 is lipoprotein, a washing liquid 70 for lipoprotein is selected as the washing liquid 70. Lipoprotein is a substance in which lipid and apoprotein are bound to each other. Thus, even in the case of measurement of lipoprotein which includes lipid and which is easily influenced by the washing liquid 70, the measurement can be suppressed from being hindered by the influence of the washing liquid 70 used in the washing process for the complex 81.

Specifically, in a case where lipoprotein is set as the target component 80, the analysis method includes a step of forming a complex that includes: lipoprotein having tagged cholesterol incorporated therein; and an antibody that binds to the lipoprotein. Thus, the function of lipoprotein can be quantitatively analyzed. Here, the "function of lipoprotein" denotes a lipid incorporating function and a lipid releasing function of lipoprotein, and is a concept that encompasses the amount of lipid that can be incorporated by lipoprotein, for example. In this case, it is possible to suppress the function of lipoprotein regarding lipid incorporation from being hindered by the washing process, and to accurately evaluate the function of lipoprotein.

In a case where the target component 80 is lipoprotein, if a washing liquid that contains no surfactant or that contains a surfactant at a concentration of less than 1.1 g/L is selected as the washing liquid 70 for lipoprotein, occurrence of structural change of the lipoprotein due to influence of the surfactant can be suppressed.

As the washing liquid 70 for lipoprotein, if a washing liquid 70 that contains a salt in an appropriate concentration range is used, even in a case where the target component 80 is lipoprotein, a high washing effect can be obtained. As a result, the background signal can be effectively reduced by the washing process.

### [Specific configuration example of analyzer]

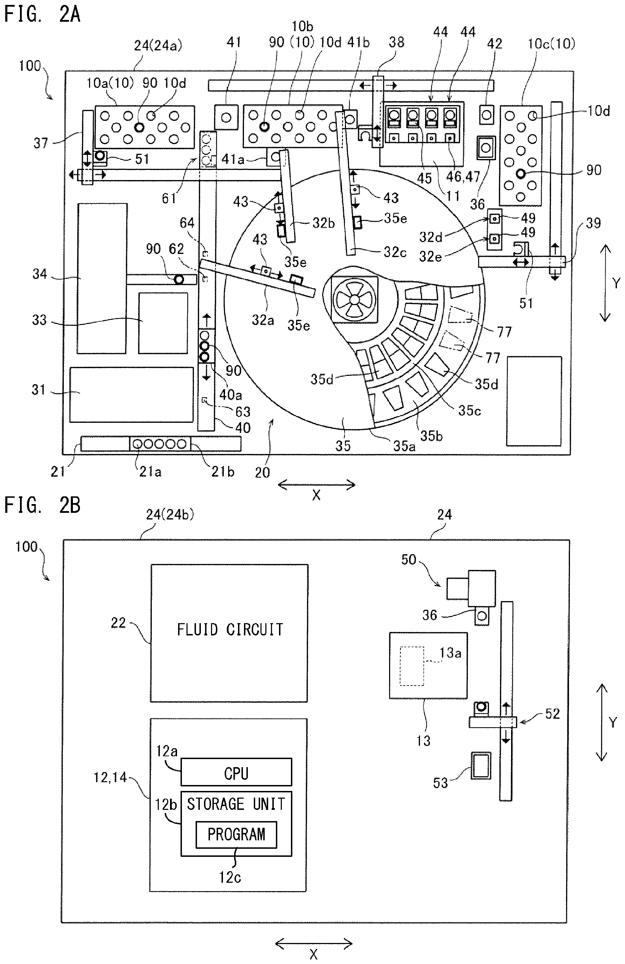

In the following, a specific configuration example of the analyzer 100 is described in detail. The respective constituents of the analyzer 100 described above are realized by the configuration as shown in FIGS. 2A and 2B.

In the example shown in FIGS. 2A and 2B, the analyzer 100 performs analysis of the target component 80 through immunoassay using antigen-antibody reaction. In the immunoassay using antigen-antibody reaction, for the purpose of improving the detection accuracy, it is desired to perform an effective washing process in order to suppress non-specific adsorption of the target component 80, reagent components, and other substances such as contaminant substances from outside, while maintaining the structure and function of the complex 81. Thus, the analyzer 100 which performs the analysis method of the present embodiment is effective in particular.

In this case, the target component is, for example, an antigen or an antibody, a protein, a peptide, or the like that is contained in blood. The analyzer 100 obtains serum as a specimen and quantitatively or qualitatively measures an antigen, an antibody, or the like contained in the specimen. The specimen may be plasma. The antigen-antibody reaction includes not only reaction between an antigen and an antibody, but also reaction using a specifically binding substance such as an aptamer. The aptamer is a nucleic acid molecule or a peptide synthesized so as to specifically binds to a specific substance.

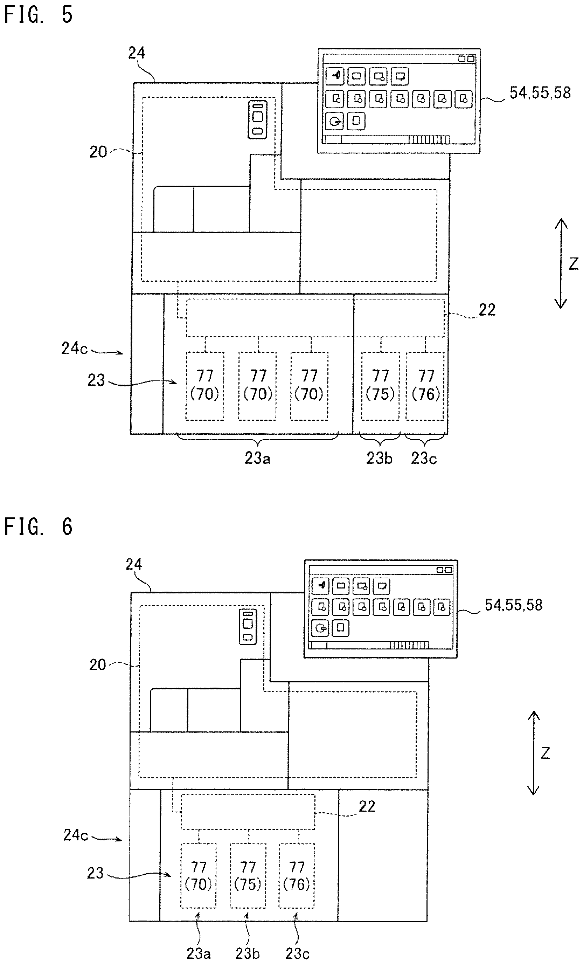

The analyzer 100 includes a measurement mechanism unit 20, a specimen transport unit 21, a fluid circuit 22, and a washing liquid storage part 23 (see FIG. 5), and these constituents are housed in a housing 24. The reaction unit 10, the washing unit 11, the controller 12, and the analysis unit 13 are provided in the measurement mechanism unit 20.

The measurement mechanism unit 20 includes a specimen dispenser 31, reagent dispensers 32a to 32e, a tip supplying unit 33, a container supplying unit 34, a reagent storage part 35, and an inter-stage transport unit 36. The measurement mechanism unit 20 includes transfer units 37, 38, 39, and 40 which transport reaction containers to these constituents.

The housing 24 has a staged structure in which a plurality of stages are provided in the up-down direction. The housing 24 includes a first stage 24a (see FIG. 2A), a second stage 24b (see FIG. 2B) below the first stage 24a, and a third stage 24c (see FIG. 5) at which the washing liquid storage part 23 and the like are disposed. The reaction container 90 is transferred between the first stage 24a and the second stage 24b by the inter-stage transport unit 36. The housing 24 may have one stage only. In the following, for convenience, the horizontal direction along the long side of the housing 24 is defined as an X direction, and the horizontal direction along the short side of the housing 24 is defined as a Y direction. The up-down direction orthogonal to the X direction and the Y direction is defined as a Z direction.

FIG. 2A shows a configuration example of the constituents provided at the first stage 24a.

The specimen transport unit 21 can transport, to a predetermined specimen aspiration position, a rack 21b in which a plurality of test tubes 21a each holding a specimen are disposed. The tip supplying unit 33 can store a large number of dispensing tips (not shown) and can supply the dispensing tips to the specimen dispenser 31. Each dispensing tip is a hollow and tubular tip component capable of holding a predetermined amount of a specimen, for example, and is configured to be disposable.

The specimen dispenser 31 has a function of dispensing a specimen into a reaction container 90. The specimen dispenser 31 can aspirate a specimen in a test tube 21a by use of a dispensing tip, and can dispense the aspirated specimen into a reaction container 90 disposed at a specimen dispensing position 63.

The container supplying unit 34 can store a plurality of the reaction containers 90 that have not been used. The container supplying unit 34 can sequentially supply the reaction containers 90 one by one to the transfer unit 40, at a reaction container supplying position 62.

The transfer unit 40 includes a holder 40a for the reaction container 90, and can move linearly in the Y direction. The transfer unit 40 can transfer the reaction container 90 to a delivering position 61, the reaction container supplying position 62, the specimen dispensing position 63, and a reagent dispensing position 64.

The reagent storage part 35 includes: a case 35a having a cylindrical shape; and reagent setting parts 35b and 35c each having an annular shape. The reagent storage part 35 is a cool box for cooling, by means of a cooling mechanism, reagents set in the case 35a having a heat insulating function.

The annular reagent setting parts 35b and 35c are disposed concentrically with each other and can rotate independently of each other. The reagent setting part 35b at the outer periphery side and the reagent setting part 35c at the inner periphery side can each hold a plurality of reagent containers 35d. In these reagent containers 35d, R1 to R3 reagents, and R11 to R13 reagents described later are held. Through rotation of the reagent setting parts 35b and 35c, a plurality of the reagent containers 35d are each located at a predetermined reagent aspiration position. Three aspiration holes 35e which each can be opened/closed are provided in the upper face of the case 35a so as to allow aspiration by the reagent dispensers 32a to 32c, respectively.

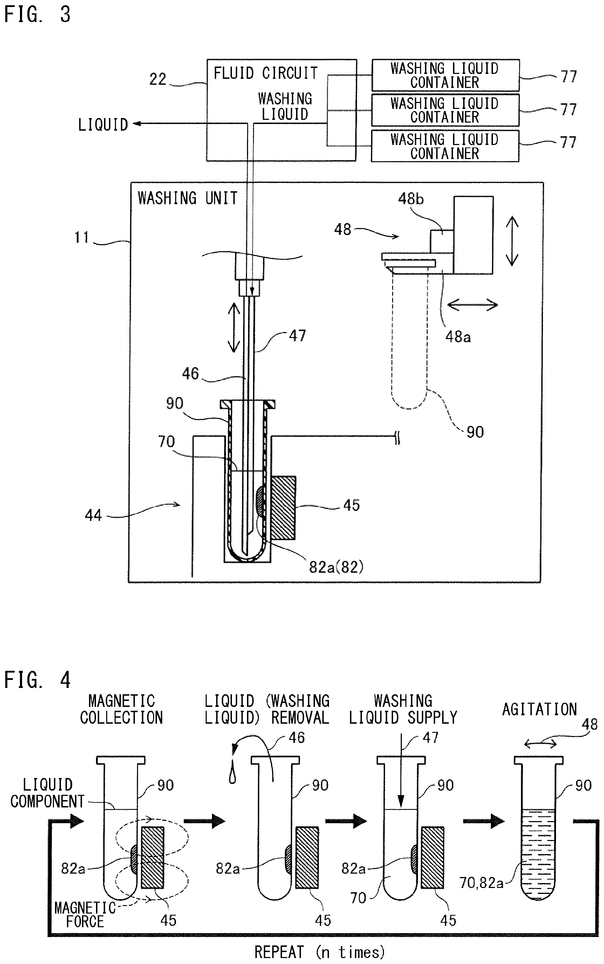

In the configuration example shown in FIG. 4, the analyzer 100 is provided with three reaction units 10a to 10c, and the three reaction units 10a to 10c are fixedly disposed. Each of the reaction units 10a to 10c includes a heater and a temperature sensor not shown, and has a function of holding the reaction container 90 and of heating the sample held in the reaction container 90 to cause the sample to react. Specifically, each of the reaction units 10a to 10c has a plurality of container holding holes 10d and can heat the samples held in the reaction containers 90 placed in the container holding holes 10d to a predetermined temperature.

The reaction unit 10a is disposed in the vicinity of the transfer unit 40. Between the reaction unit 10a and the reaction unit 10b, a magnetic collection port 41 is provided that holds a reaction container 90 in a holding hole and that collects the magnetic particles 82a in the sample in the reaction container 90 by means of a magnet. At the magnetic collection port 41, the reaction container 90 can be delivered between the transfer unit 37 and the transfer unit 38.

The reaction unit 10b is located between the reaction unit 10a and the washing unit 11. A dispensing port 41a is provided between the reaction unit 10b and the reagent storage part 35. A dispensing port 41b is provided between the reaction unit 10b and the washing unit 11. The reaction unit 10c is located at an X2 direction side relative to the washing unit 11. A relay unit 42 and the inter-stage transport unit 36 are provided between the washing unit 11 and the reaction unit 10c.

The reagent dispenser 32a to the reagent dispenser 32c each perform a process of dispensing a reagent into a reaction container 90. The reagent dispenser 32a can move a pipette 43 for aspirating and discharging a reagent, between the aspiration hole 35e and the reagent dispensing position 64. The pipette 43 aspirates a reagent from a reagent container 35d in the reagent storage part 35, and dispenses the reagent into a reaction container 90 transferred to the reagent dispensing position 64.

The reagent dispenser 32b can move a pipette 43 between the aspiration hole 35e and the dispensing port 41a. The pipette 43 aspirates a reagent from a reagent container 35d in the reagent storage part 35, and dispenses the reagent into a reaction container 90 transferred to the dispensing port 41a.

The reagent dispenser 32c can move a pipette 43 between the aspiration hole 35e and the dispensing port 41b. The pipette 43 aspirates a reagent from a reagent container 35d in the reagent storage part 35, and dispenses the reagent into a reaction container 90 transferred to the dispensing port 41b.

As described above, the washing unit 11 has a function of supplying the washing liquid 70 into the reaction container 90 and performing a BF separation process which is a washing process for the complex 81. The washing unit 11 includes a plurality of washing ports 44 each capable of having a reaction container 90 disposed therein. The washing unit 11 includes: a magnetic collection part 45 which captures the complex 81 in the reaction container 90 by magnetic force; an aspiration part 46 which removes the liquid component in the reaction container 90; and a discharging part 47 which discharges the washing liquid 70. Each washing port 44 is provided with the magnetic collection part 45, the aspiration part 46, and the discharging part 47. The magnetic collection part 45 is implemented by a permanent magnet, for example. The magnetic collection part 45 may be an electromagnet. In the configuration example shown in FIGS. 2A and 2B, four washing ports 44 are provided.

The relay unit 42 has a holding hole that can hold a reaction container 90. At the relay unit 42, the reaction container 90 is delivered between the transfer unit 38 and the transfer unit 39.

The reagent dispenser 32d and the reagent dispenser 32e are disposed side by side, and each include a reagent nozzle 49. The reagent dispenser 32d and the reagent dispenser 32e discharge an R4 (R14) reagent and an R5 (R15) reagent through the reagent nozzles 49 into a reaction container 90, respectively.

The inter-stage transport unit 36 has a holding hole that can hold a reaction container 90. The inter-stage transport unit 36 is raised/lowered between the first stage 24a and the second stage 24b by a raising/lowering device 50.

The transfer units 37, 38, and 39 each have a function of holding a reaction container 90 and transporting the reaction container 90 to each constituent. Each of the transfer units 37, 38, and 39 is an orthogonal robot mechanism that can move along three orthogonal axes, i.e., two horizontal axes and one vertical axis. The structures of the transfer units 37, 38, and 39 are basically the same, and can employ known configurations.

Each of the transfer units 37 to 39 has a catcher 51 which grips a reaction container 90. Each of the transfer units 37 to 39 can take out reaction containers 90 one by one by the catcher 51 and can transport the reaction container 90 to any position within a movable range. For example, the transfer unit 38 transfers a reaction container 90 disposed in the reaction unit 10b to any of the washing ports 44 of the washing unit 11.

### (Second stage)

FIG. 2B shows a configuration example of each constituent disposed on the second stage 24b.

On the second stage 24b of the housing 24, the analyzer 100 includes the analysis unit 13, the raising/lowering device 50, a container transport unit 52, a container discard hole 53, the controller 12, and the fluid circuit 22.

The container transport unit 52 transports a reaction container 90 among the inter-stage transport unit 36 having been lowered to the second stage 24b, the analysis unit 13, and the container discard hole 53.

The analysis unit 13 includes a photodetector 13a such as a photomultiplier. The analysis unit 13 obtains, by means of the photodetector 13a, light generated in the reaction process between the luminescent substrate and the labeled substance 83 binding to the target component 80, thereby to quantitatively analyze the target component 80 contained in the sample.

The controller 12 includes a CPU 12a, a storage unit 12b, and the like. The CPU 12a executes a control program 12c stored in the storage unit 12b, thereby functioning as the controller of the analyzer 100. The controller 12 controls operation of each constituent of the analyzer 100 described above. In the example shown in FIGS. 2A and 2B, the setting unit 14 is implemented by the same hardware as the controller 12. That is, the CPU 12a executes the control program 12c stored in the storage unit 12b, thereby functioning as the setting unit 14 of the analyzer 100. In other words, in the example shown in FIGS. 2A and 2B, the controller 12 is configured to function also as the setting unit 14.

The analyzer 100 includes: a display unit 54 (see FIG. 5) which displays various types of screens; and an input unit 55 (see FIG. 5) which receives operation inputs. The input unit 55 is a touch panel, for example, and is integrated with the display unit 54. The input unit 55 may be an input device such as a keyboard or a mouse.

The fluid circuit 22 is in fluid connection with constituents such as the washing unit 11, the specimen dispenser 31, and the reagent dispensers 32a to 32e. The fluid circuit 22 has a function of supplying pressure associated with aspiration/discharge operation of a specimen and a reagent, and a function of supplying the washing liquid 70 in association with washing operation in the washing unit 11. The fluid circuit 22 can supply, into the apparatus, a later- described washing liquid 75 for washing a liquid distribution channel, thereby to wash the liquid distribution channel. The fluid circuit 22 includes, for example: supply channels for supplying liquids; an air pressure source for applying pressure to each supply channel to send a liquid; a valve for switching channels of liquids; a liquid chamber for measuring the amount of a liquid; and the like. The fluid circuit 22 is connected through connection tubes and the like to various types of washing liquid containers 77 (see FIG. 5) disposed in the washing liquid storage part 23 (see FIG. 5), for example, and can aspirate the washing liquids 70 from the washing liquid containers 77.

### (Washing unit)

FIG. 3 shows a configuration example of the washing unit 11. The aspiration part 46 includes an aspiration tube which aspirates unnecessary components such as liquid containing unreacted labeled substance 83, for example. The discharging part 47 includes a discharge tube which discharges the washing liquid 70 into a reaction container 90, for example.

When the magnetic particles 82a are used as the carrier 82, the washing unit 11 captures, by means of the magnetic collection part 45, the magnetic particles 82a having formed thereon the complex 81 that includes the target component 80 and the labeled substance 83. The washing unit 11 is configured to be able to hold the reaction container 90 in the washing port 44, and the magnetic collection part 45 is disposed in the vicinity of the reaction container 90 held in the washing port 44. FIG. 3 shows a configuration example in which: the magnetic collection part 45 is disposed at a position close to the side face of the reaction container 90 disposed in the washing port 44 and magnetically collects the magnetic particles 82a to the inner side face of the reaction container 90.

The washing unit 11 includes agitation parts 48 provided for the respective washing ports 44, for example. Each agitation part 48 includes: a catcher 48a movable in such a manner as to grip and lift the reaction container 90 held in the washing port 44; and a drive source 48b which applies vibration to the reaction container 90 gripped by the catcher 48a.

### <Washing process>

The washing process (BF separation) includes a step of magnetically collecting the complex 81; a step of supplying the washing liquid 70 to be used in the washing process; and a step of removing the washing liquid 70. Thus, through the supply and removal of the washing liquid 70, components unnecessary for the measurement other than the carrier 82 that includes the complex 81 can be effectively removed.

For example, in FIG. 4, the washing process includes: (a) magnetic collection step of collecting the complex 81 in the reaction container 90; (b) removal step of removing the washing liquid 70 in the reaction container 90; (c) supply step of supplying the washing liquid 70 into the reaction container 90; and (d) agitation step in a magnetic collection cancelled state. The washing process is performed by the controller 12 controlling the washing unit 11.

First, in (a) magnetic collection step, the magnetic collection part 45 magnetically collects the magnetic particles 82a in the reaction container 90 disposed in the washing port 44. Next, in (b) removal step, the aspiration part 46 aspirates the liquid component from the reaction container 90 in a captured state in the washing port 44, and removes the liquid component from the reaction container 90. After the washing liquid 70 is supplied into the reaction container 90, the washing liquid 70 is removed in the removal step. At this time, the magnetic particles 82a carrying the complex 81 remain, being magnetically collected in the reaction container 90. Next, in (c) supply step, the discharging part 47 supplies a predetermined amount of the washing liquid 70 into the reaction container 90 in a captured state in the washing port 44. At this time, the controller 12 controls the fluid circuit 22 to cause the washing liquid 70, which has been selected in accordance with the target component 80, to be discharged from the discharging part 47. Next, in (d) agitation step, the agitation part 48 takes out the reaction container 90 from the washing port 44, and applies vibration to agitate the sample containing the magnetic particles 82a. By the reaction container 90 being taken out of the washing port 44, magnetic collection by the magnetic collection part 45 is canceled. Through the vibration, the magnetic particles 82a are diffused in the washing liquid 70.

In the washing process, the controller 12 controls the washing unit 11 so as to repeat the steps (a) to (d) described above a predetermined multiple times (n times). As a result, unnecessary components non-specifically attached to the reaction container 90 and the magnetic particles 82a are removed. After the steps (a) to (d) are repeated a predetermined number of times, the washing liquid 70 is lastly removed from the reaction container 90 in (b) removal step, and the washing process ends.

In the example shown in FIG. 4, for example, in accordance with the target component 80, a washing method to be used in the washing process is selected, and the complex 81 including the target component 80 is washed by the selected washing method. In this case, the washing process can be executed, not only with use of the washing liquid 70 that is appropriate for the target component 80, but also with an appropriate washing method selected. Accordingly, the washing liquid 70 and the washing method can be optimized. Thus, a washing process in which the influence of the washing liquid 70 is more effectively suppressed and a washing process in which the background signal is more effectively reduced can be performed.

The content of selection of a washing method includes, for example, any of magnetic collection time period of the complex 81, strength of magnetic collection force, supply amount of the washing liquid 70, and repetition number of times of the washing method.

The magnetic collection time period of the complex 81 is a time period for which magnetic collection of the magnetic particles 82a by the magnetic collection part 45 is continued in (a) magnetic collection step, and corresponds to a waiting time period from the reaction container 90 is disposed in the washing port 44 until (b) removal step is performed.

The strength of magnetic collection force is the strength of magnetic force to be acted by the magnetic collection part 45 on the magnetic particles 82a in the reaction container 90. For example, in the case of the configuration example shown in FIG. 4, the strength of magnetic collection force can be controlled by changing the distance between the magnet and the reaction container 90, or by the number or the kind of magnet disposed at the washing port 44. When the magnetic collection part 45 is provided with an electromagnet, the strength of the magnetic force can be electrically controlled. Through selection of the magnetic collection time period and the strength of magnetic collection force, the magnetic collection step can be optimized in such a case where the kind of the magnetic particles 82a to be used is different according to the target component 80.

The supply amount of the washing liquid 70 is the amount of the washing liquid 70 to be discharged into the reaction container 90 after the removal step. The repetition number of times of the washing method specifies how many times the steps (a) to (d) are repeated. As a result of optimization of the washing liquid 70, it becomes possible to reduce the time period necessary for the washing process by employing a fewer repetition number of times, and, with respect to a target component 80 for which the strength of the background signal tends to increase, it becomes possible to optimize the removing effect of unnecessary components by the washing process, by increasing the repetition number of times in particular.

In a case where a washing method is selected, for example, the storage unit 12b of the controller 12 is previously caused to store information which mutually associates target components 80, washing liquids 70 to be used, and washing methods to be performed. Accordingly, a washing liquid 70 and a washing method that are optimized in accordance with a target component 80 are selected.

### (Selection of washing liquid)

Next, configuration for selecting the washing liquid 70 is described. In the examples shown in FIG. 5 to FIG. 7, before performing measurement of a plurality of target components 80, an appropriate washing liquid 70 is selected in accordance with a target component 80 from among a plurality of washing liquids 70, and the selected washing liquid 70 is supplied to the washing unit 11. For selection of the washing liquid 70, various configurations can be employed.

In the configuration example shown in FIG. 5, a washing liquid 70 to be used is selected from among a plurality of kinds of washing liquids 70 disposed in the analyzer 100. Specifically, the analyzer 100 includes the washing liquid storage part 23 in which a plurality of the washing liquids 70 are stored. The controller 12 selects a washing liquid 70 to be used in the washing process, from among the washing liquids 70 stored in the washing liquid storage part 23. Accordingly, for example, different from a case where the washing liquid 70 is supplied from outside the apparatus, the analyzer 100 can singly select a washing liquid 70 and perform the washing process. As a result, the apparatus configuration can be simplified, and analysis of the target component 80 can be efficiently performed.

For example, in FIG. 5, the washing liquid storage part 23 includes: a first storage part 23a which stores the washing liquid 70 to be used in the washing process for the complex 81; a second storage part 23b which stores the washing liquid 75 for washing the liquid distribution channel; and a third storage part 23c which stores a washing liquid 76 for washing the pipettes 43 of the reagent dispensers 32a to 32e.

The washing liquid storage part 23 may be configured to allow the container of the washing liquid 70 to be disposed as is, or may be provided with a chamber which holds the washing liquid 70. In FIG. 5, the washing liquid storage part 23 is configured to allow a plurality of the washing liquid containers 77 to be disposed therein. In the first storage part 23a, a plurality of the washing liquid containers 77 respectively containing different kinds of washing liquids 70 are disposed, and are each connected to the fluid circuit 22.

In this configuration example, the controller 12 selects a washing liquid 70 stored in the first storage part 23a, as the washing liquid 70 to be used in the washing process. Accordingly, from among the washing liquids 70 for the washing process for the complex 81 and stored in the first storage part (23a), an appropriate washing liquid 70 can be easily selected to be used in the washing process.

In a case where a plurality of washing liquids respectively having different usages are stored in the analyzer 100, a washing liquid for a usage other than for the washing process for the complex 81 may be used in the washing process for the complex 81.

In the configuration example shown in FIG. 6, the first storage part 23a, the second storage part 23b, and the third storage part 23c are each configured to allow one washing liquid container 77 to be disposed therein. Each washing liquid container 77 is connected to the fluid circuit 22.

In the configuration example shown in FIG. 6, the controller 12 selects the washing liquid 75 stored in the second storage part 23b, as the washing liquid 70 to be used in the washing process. That is, from the washing liquid 70 being for the washing process for the complex 81 and stored in the first storage part 23a, and the washing liquid 75 being for washing the liquid distribution channel and stored in the second storage part 23b, the controller 12 selects a washing liquid to be used in the washing process for the complex 81 in accordance with the target component 80.

Thus, the washing liquid 75 for washing the liquid distribution channel can be used as the washing liquid 70 to be used in washing the complex 81. That is, different from a case where a plurality of kinds of the washing liquids 70 for the washing process for the complex 81 are prepared, increase in the number of kinds of the washing liquids 70 stored in the analyzer 100 can be suppressed by using the washing liquid 75 for washing the liquid distribution channel.

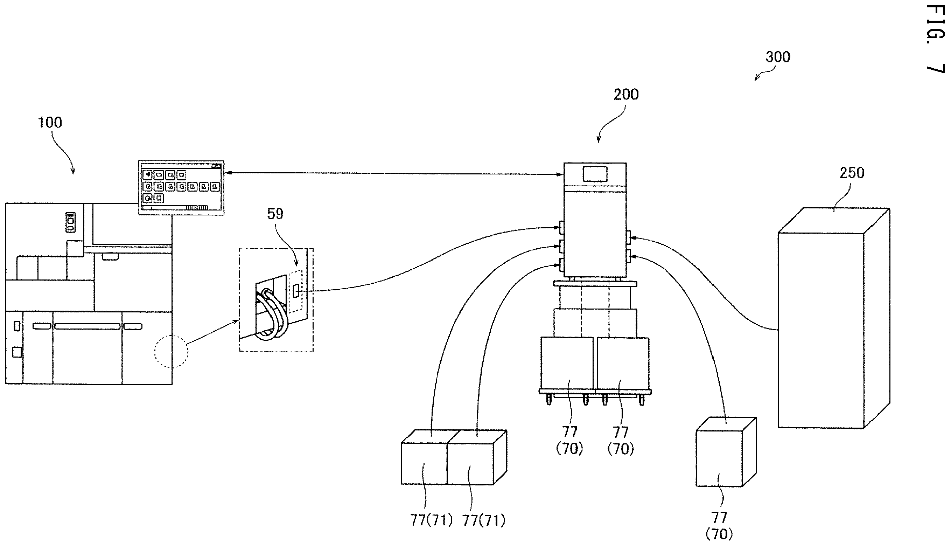

The washing liquid 70 to be used in the washing process for the complex 81 may be configured to be supplied from outside of the analyzer 100. FIG. 7 shows a configuration example of a specimen analysis system 300 including the analyzer 100 and a washing liquid supply apparatus 200.

The analyzer 100 causes the complex 81 including a target component 80 contained in a specimen to be formed on the carrier 82 in a reaction container 90, and supplies the washing liquid 70 into reaction container 90 to wash the complex 81. The washing liquid supply apparatus 200 is in fluid connection with the analyzer 100, and stores a plurality of the washing liquids 70. The washing liquid supply apparatus 200 is configured to supply the analyzer 100 with the washing liquid 70 selected in accordance with the target component 80. Thus, the analyzer 100 obtains from the washing liquid supply apparatus 200 the washing liquid 70 selected in accordance with the target component 80, and performs the washing process for the complex 81.

For example, the analyzer 100 includes a connection hole 59 for introducing the washing liquid 70 from outside the apparatus, and is connected to a supply hole of the washing liquid supply apparatus 200 through a connection tube or the like.

The washing liquid supply apparatus 200 is in fluid connection with a plurality of the washing liquid containers 77, can selectively aspirate a washing liquid 70 from the washing liquid containers 77, and can send the aspirated washing liquid 70 to the analyzer 100. The washing liquid containers 77 connected to the washing liquid supply apparatus 200 contain different kinds of washing liquids 70, respectively.

Thus, for each target component 80, a washing liquid 70 suitable for the measurement of the target component 80 can be supplied from the washing liquid supply apparatus 200 to the analyzer 100, to perform the washing process for the complex 81. That is, in a case where measurement of a plurality of target components 80 is performed, even with respect to a specific target component 80 for which the measurement is hindered by a general washing liquid 70, the measurement can be performed with use of a washing liquid 70 that does not hinder the measurement. Thus, even in a case where measurement of a plurality of target components 80 is performed by the analyzer 100, the measurement can be suppressed from being hindered by influence of the washing liquid 70 used in the washing process for the complex 81. Also with respect to a general target component 80 for which a common washing liquid 70 is conventionally used, the measurement accuracy can be further improved by selecting an appropriate washing liquid 70, and supplying the selected washing liquid 70 from the washing liquid supply apparatus 200 to the analyzer 100 to perform the washing process.

The washing liquid 70 may be selected from the reagent storage part 35. For example, in FIG. 2A, in the reagent storage part 35, washing liquid containers 77 (see chain double-dashed lines) are disposed in addition to various types of reagent containers 35d. The controller 12 selects a washing liquid 70 to be used in the washing process from among the washing liquids 70 stored in the reagent storage part 35. In this manner, by use of the reagent storage part 35 for storing reagents, washing liquids 70 can also be stored. As a result, when compared with a case where a storage part for storing a plurality of kinds of washing liquids 70 is separately provided, without making the apparatus configuration significantly different from conventional apparatus configurations, a large number of kinds of washing liquids 70 can be easily stored and a washing liquid 70 can be selected in accordance with the target component 80.

### <Preparation of washing liquid>

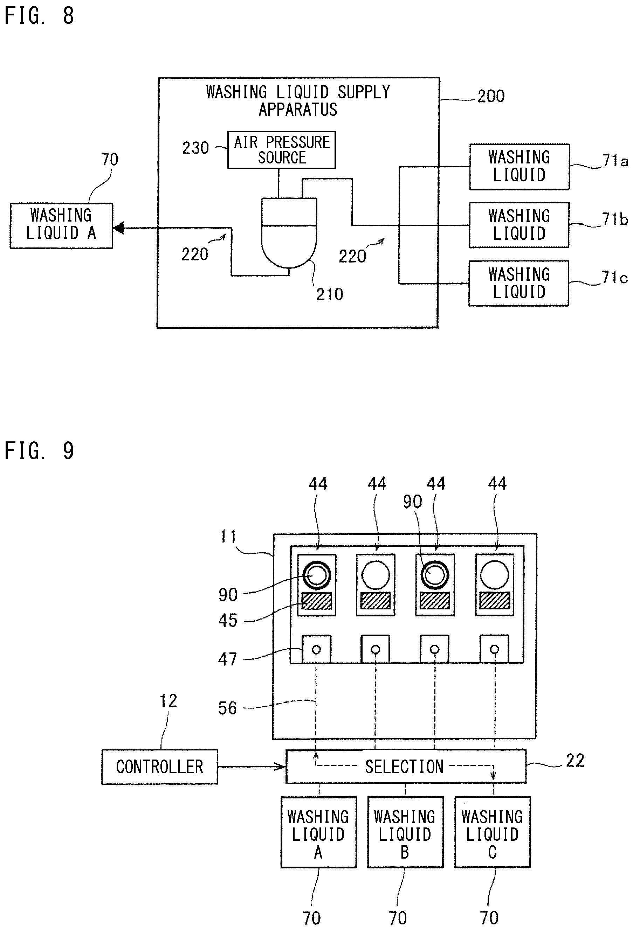

The washing liquid 70 that is selected may not be the one having been prepared in advance so as to have a predetermined composition, but may be prepared by use of various components. In the configuration example shown in FIG. 8, from among a plurality of washing liquids 71a to 71c, one washing liquid and another washing liquid are mixed together, to prepare a washing liquid 70 to be used in the washing process. Accordingly, from among the plurality of kinds of washing liquids 71a to 71c prepared in advance, a washing liquid 70 having a more diverse composition can be prepared. Thus, the kinds of selectable washing liquids 70 can be easily increased.

Specifically, a washing liquid 70 is prepared by mixing two or three types of the washing liquid 71a, the washing liquid 71b and the washing liquid 71c at their respective predetermined proportions. Each of the washing liquids 71a to 71c can include, for example: water and a buffer serving as a solvent; a surfactant and a salt as a washing component; another supplementary agent and another adjusting agent; and the like. For example, the washing liquid supply apparatus 200 is connected to a pure water generation apparatus 250 (see FIG. 7), and may prepare a washing liquid 70 by mixing pure water with a high concentration washing liquid 71 contained in a washing liquid container 77. In this case, the washing liquid supply apparatus 200 includes: a mixing part 210 for mixing the washing liquid 71; a passage part 220 which connects the mixing part 210 to the washing liquid container 77, an air pressure source 230 which sends liquid through the passage part 220; various types of valves (not shown) for switching the passages; and the like.

### (Method for supplying washing liquid)

In a case where the washing unit 11 includes a plurality of the washing ports 44 as in the configuration example shown in FIG. 2A, the washing unit 11 is configured to be able to switch, for each washing port 44, the washing liquid 70 among a plurality of kinds, to be supplied.

Specifically, as shown in FIG. 9, the controller 12 performs control of selecting a washing liquid 70 to be supplied to the reaction container 90 in each washing port 44. By controlling the fluid circuit 22, the controller 12 individually selects the kind of the washing liquid 70 to be supplied from the discharging part 47 in each of the plurality of the washing ports 44. In FIG. 9, a supply channel 56 for a washing liquid 70 selected from among a washing liquid A to a washing liquid C, and the discharging part 47 are connected to each other. In this case, without increasing the number of the washing ports 44, various types of the washing liquids 70 can be selected and supplied. In the configuration example shown in FIG. 9, a plurality of kinds of the washing liquids 70 are sent through the common supply channel 56.

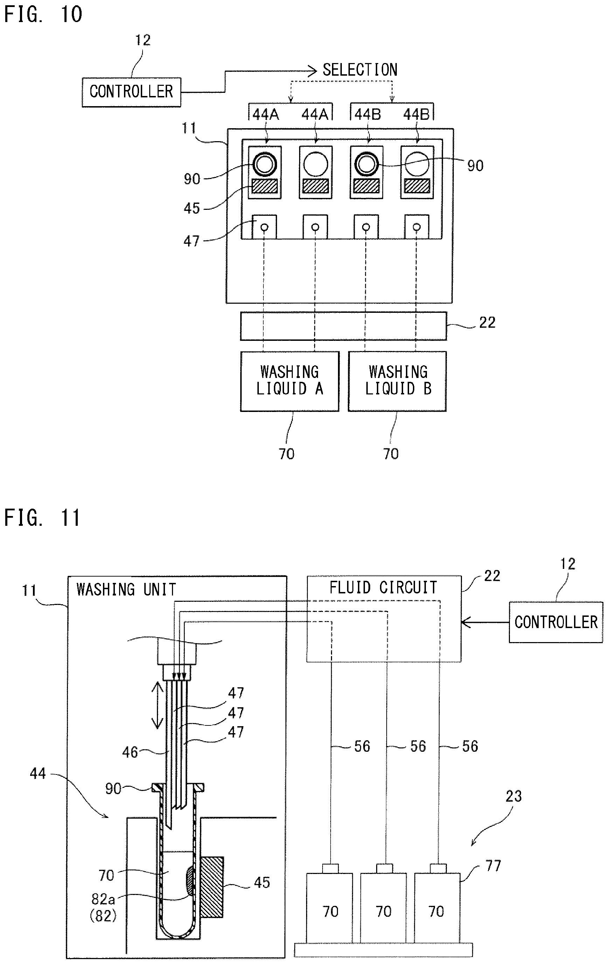

For example, in the configuration example shown in FIG. 10, the washing unit 11 is configured such that only one-specific kind of washing liquid 70 is supplied in each of the washing ports 44. In addition, the washing unit 11 is configured such that a plurality of kinds of the washing liquids 70 are supplied from separate washing ports 44. FIG. 10 shows an example in which: in two washing ports 44A, a washing liquid A is supplied, and in two washing ports 44B, a washing liquid B is supplied.

In this case, the controller 12 performs control of disposing a reaction container 90 at a washing port 44 to which the selected washing liquid 70 is to be supplied. That is, the controller 12 selects a washing port 44 in which to set a reaction container 90 holding a target component 80, from among a plurality of washing ports 44A and 44B, thereby selecting a washing liquid 70 to be supplied to the reaction container 90. The controller 12 controls the transfer unit 38, for example, to cause a reaction container 90 to be set at the selected washing port 44. In this case, the kind of the washing liquid 70 supplied at the individual washing port 44 can be fixed, and thus, during supply of the washing liquids 70 to the reaction container 90, mixing of different kinds of washing liquids 70 can be prevented.

In the configuration example shown in FIG. 11, each individual washing port 44 is provided with a plurality of the supply channels 56 for supplying the washing liquids 70 from the washing liquid storage part 23 to the washing unit 11. In this case, in accordance with the selected washing liquid 70, the controller 12 performs control of selecting a separate supply channel 56 to send the liquid. Thus, the supply channels 56 for a plurality of kinds of the washing liquids 70 can be individually provided. As a result, during supply of the washing liquids 70 to the reaction container 90, mixing of different kinds of washing liquids 70 can be prevented.

FIG. 11 shows an example in which: three systems of the supply channels 56 are provided so as to correspond to three kinds of washing liquids 70; and the supply channels 56 distribute specific washing liquids 70, respectively. With respect to the washing port 44, three discharging parts 47 are connected to the separate supply channels 56, respectively. By switching the fluid circuit 22, the controller 12 causes the selected washing liquid 70 to be supplied to the reaction container 90 through the corresponding supply channel 56.

### (Selection of washing liquid)

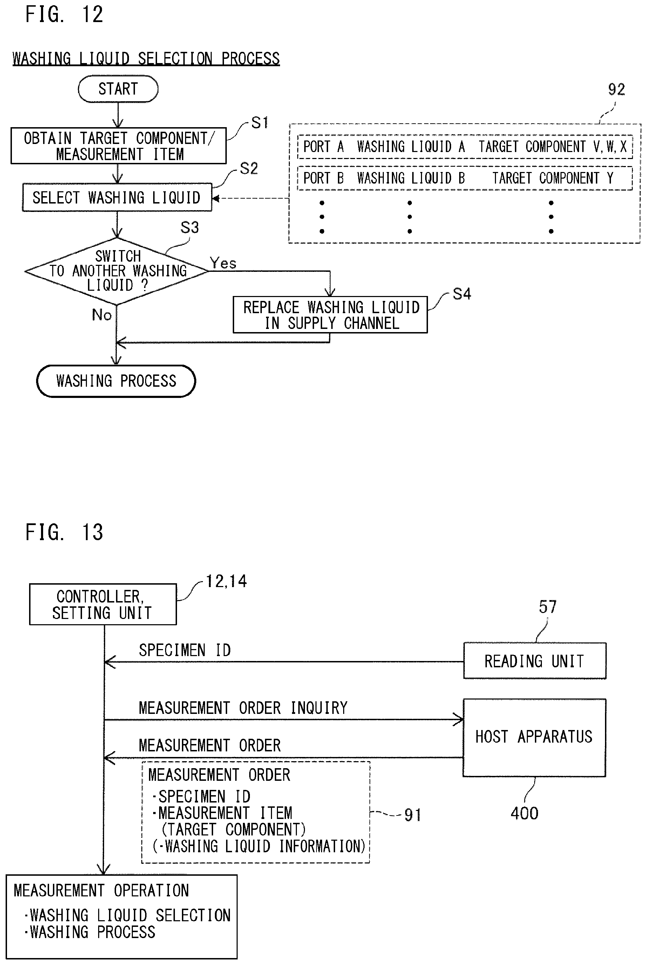

FIG. 12 shows an example of control of selection of the washing liquid 70 performed by the controller 12.

In step S1, the controller 12 obtains a target component 80 or a measurement item of a specimen to be subjected to the washing process performed by the washing unit 11.

In step S2, the controller 12 selects a washing liquid 70 in accordance with the obtained target component 80 or measurement item. For example, the storage unit 12b has stored therein information 92 which associates the connection port (port A, port B, ...) of the fluid circuit 22, the kind (washing liquid A, washing liquid B, ...) of the washing liquid 70, the target component 80 or measurement item (target component V, W, X, target component Y, ...) with one another. On the basis of the obtained target component 80 or measurement item, the controller 12 specifies a washing liquid 70 associated with the target component 80 or measurement item, and selects a connection port connected to the washing liquid container 77 of the specified washing liquid 70. The fluid circuit 22 supplies the washing liquid 70 to the washing unit 11 through the designated port.

In this manner, in the example shown in FIG. 12, the controller 12 selects the washing liquid 70 to be used in the washing process for the complex 81 in accordance with the target component 80. Accordingly, there is no need to receive a selection command regarding a washing liquid 70 from an external apparatus, or to receive selection of a washing liquid 70 by an input operation performed by the user, for example, and the analyzer 100 can singly select an appropriate washing liquid 70 to perform the washing process.

In step S3, the controller 12 determines whether to switch the washing liquid 70 to be used in the washing port 44 at which the washing process is performed, to another kind of washing liquid 70. When switching to another washing liquid 70, the controller 12 performs, in step S4, control of causing the washing liquid 70 remaining in the discharging part 47 and the supply channel 56 of the washing port 44 to be replaced with the selected washing liquid 70 to wash the discharging part 47 and the supply channel 56.

Thus, in the example shown in FIG. 12, when switching one washing liquid 70 to another washing liquid 70, the controller 12 performs control of washing, with the another washing liquid 70, the supply channel 56 through which the one washing liquid 70 has been sent. Thus, in a case where the washing liquids 70 are supplied through a common supply channel 56 as in, for example, a configuration example shown in FIG. 9, one previously-used washing liquid 70 can be prevented from being supplied to the reaction container 90 in a state of being mixed with another subsequently-used washing liquid 70. In the configurations as shown in FIG. 10 and FIG. 11 where the washing liquids 70 are supplied through the individual supply channels 56, respectively, the supply channels 56 need not be washed with the washing liquids 70.

When the washing liquid 70 to be used is not changed in step S3, or after step S4, the controller 12 advances to the washing process.

### (Method for specifying target component)

FIG. 13 shows one example of a method for specifying a target component 80.

In FIG. 13, a step of obtaining information that specifies a target component 80, a step of selecting a washing process on the basis of the obtained information that specifies the target component 80 are performed. That is, the setting unit 14 obtains the information that specifies a target component 80, and the controller 12 selects a washing process on the basis of the obtained information that specifies the target component 80. The information that specifies a target component 80 is the kind of the specimen, the specimen ID, the measurement order, and the like, for example. By obtaining the information that specifies a target component 80, it is possible to easily select an appropriate washing liquid 70 in accordance with the target component 80 to perform the washing process.

In the example shown in FIG. 13, the setting unit 14 reads out a specimen ID attached to the specimen container set in the analyzer 100, by means of a reading unit 57 such as a bar code reader, and makes an inquiry to a host apparatus 400 which manages measurement orders 91. As a response to the inquiry, the host apparatus 400 transmits to the setting unit 14 a measurement order 91 that corresponds to the specimen ID.

Accordingly, the setting unit 14 obtains the measurement order 91 for the specimen set in the analyzer 100. In the measurement order 91, for each specimen, a measurement item is set so as to be associated with the specimen ID. All the specimens dispensed into reaction containers 90 in the analyzer 100 are managed so as to be associated with specimen IDs, and each measurement operation is performed in accordance with the measurement order 91. Thus, on the basis of the measurement order 91, the target component 80 in each reaction container 90 to be subjected to a washing process is specified. The setting unit 14 delivers the measurement order 91 to the controller 12.

The controller 12 selects a washing process in accordance with the measurement item in the measurement order 91. For example, on the basis of the measurement item, and the information 92 which associates the target component 80 with the kind of the washing liquid 70 shown in FIG. 12, the controller 12 selects a washing liquid 70 to be used. Thus, by use of the measurement order 91 obtained for the analyzer 100 to perform measurement, selection of a washing process such as the kind and the like of the washing liquid 70 can also be performed. Thus, there is no need to separately provide the analyzer 100 with information for selecting a washing process, and an appropriate washing process in accordance with the target component 80 can be easily selected.

In the measurement order 91, the kind of the washing process may be designated. For example, the measurement order 91 may include in advance information of the washing process such as the washing liquid 70 to be used. In this case, the controller 12 performs control of selecting the washing process designated in the measurement order 91 to perform the washing process.

### (Reception of selection input)

Selection of a washing process may be performed in the form of receiving an operation input performed by the user, other than in the form of being automatically performed by the controller 12 in accordance with the target component 80.

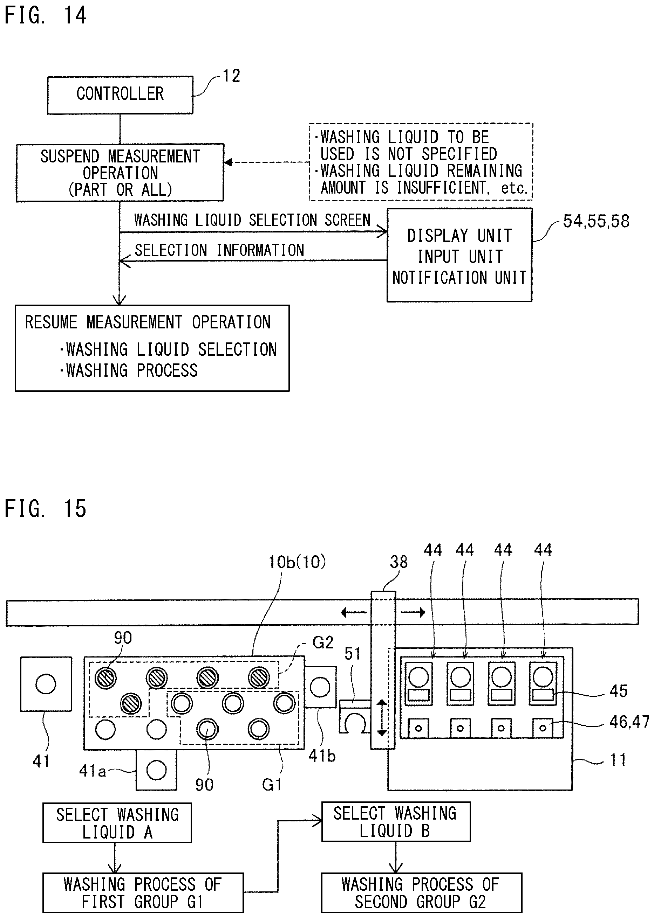

In the example shown in FIG. 14, on the basis of information received through the input unit 55, the controller 12 selects a washing liquid 70 to be used in the washing process. Thus, by allowing, through the input unit 55, the user to determine the washing liquid 70 to be used, or by the controller 12 determining a washing liquid 70 to be used on the basis of the information inputted by the user, it is possible to cause the determined washing liquid 70 to be supplied to the washing unit 11. As a result, an appropriate washing liquid 70 can be easily selected.

For example, in such a case where the washing liquid 70 to be used cannot be selected (specified) or where the remaining amount of the selected washing liquid 70 is insufficient after a reaction container 90 to be subjected to a washing process has been transferred to the washing unit 11, the controller 12 suspends part or all of the measurement operation of the analyzer 100. The controller 12 may suspend part or all the measurement operation every time a washing process is performed.

In the suspension of the measurement operation, the entirety of the apparatus may be stopped, for example. Alternatively, for example, only with respect to samples not yet subjected to the washing process, the measurement operation may be suspended, and with respect to samples having been subjected to the washing process, subsequent processes may be continued.

In this case, for example, the controller 12 suspends the measurement when the washing process is to be performed, and controls a notification unit 58 so as to make notification that urges an input to be made through the input unit 55. The notification unit 58 may be, for example, a speaker that performs sound notification, or the display unit 54 that performs notification by displaying a message. Accordingly, when a washing liquid 70 needs to be selected, notification can be provided to the user through the notification unit 58, and thus, the washing process can be more reliably performed with an appropriate washing liquid 70 selected.

For example, the controller 12 causes the display unit 54 to display the specimen ID, information of the target component 80, and choices of the washing liquid 70 to be used, thereby making notification for urging the user to perform an input through the input unit 55. The user selects, through the input unit 55, an appropriate washing liquid 70 from among the choices displayed on the display unit 54, for example. When the washing liquid 70 is insufficient, the washing liquid container 77 in the washing liquid storage part 23 is replaced.

After the washing liquid 70 according to the target component 80 is selected, the controller 12 resumes the measurement operation of the analyzer 100 that has been stopped. The controller 12 causes the selected washing liquid 70 to be supplied to the washing unit 11, to perform the washing process. Thus, in the example shown in FIG. 14, with the measurement operation suspended, the user is allowed to select an appropriate washing liquid 70. Also when the washing liquid 70 has become insufficient, supply of an inappropriate washing liquid 70 can be inhibited, and stop of supply of the washing liquid 70 can be inhibited.

### (Skipping process of measurement operation)

In the example shown in FIG. 15, in accordance with the target component 80, part or all of the measurement operation regarding the target component 80 by the analyzer 100 is skipped, and after a washing liquid 70 according to the target component 80 is selected, the skipped measurement operation regarding the target component 80 is performed.

For example, for easy explanation, a case is considered in which reaction containers 90 of a first group G1 containing a specific target component 80 that corresponds to the washing liquid A, and reaction containers 90 of a second group G2 containing another target component 80 that corresponds to the washing liquid B are present. In FIG. 15, the reaction containers 90 of the second group G2 are expressed with hatching, for convenience.

In this case, for example, the controller 12 selects the washing liquid A first, and controls the washing unit 11 and the transfer unit 38 so as to perform the washing process for the complex 81 in the reaction containers 90 of the first group G1 by use of the washing liquid A. Then, until the washing process on the reaction containers 90 of the first group G1 is completed, the washing process on the reaction containers 90 of the second group G2 is skipped and the reaction containers 90 of the second group G2 are held in the reaction unit 10 (here, the reaction unit 10b).

After the washing process on a certain number of reaction containers 90 of the first group G1 has been completed, the controller 12 selects the washing liquid B and controls the washing unit 11 so as to perform, by use of the washing liquid B, the washing process for the complex 81 in the reaction containers 90 of the second group G2 being held in the reaction unit 10.

Thus, after the measurement operation regarding the target component 80 using the washing liquid A belonging to the first group G1 has been performed, the measurement operation using another washing liquid B belonging to the second group G2 that has been skipped can be performed collectively. As a result, the number of times of switching the washing liquids 70 can be reduced, and thus, analysis of the target component 80 can be efficiently performed.

### (Specific example of measurement)

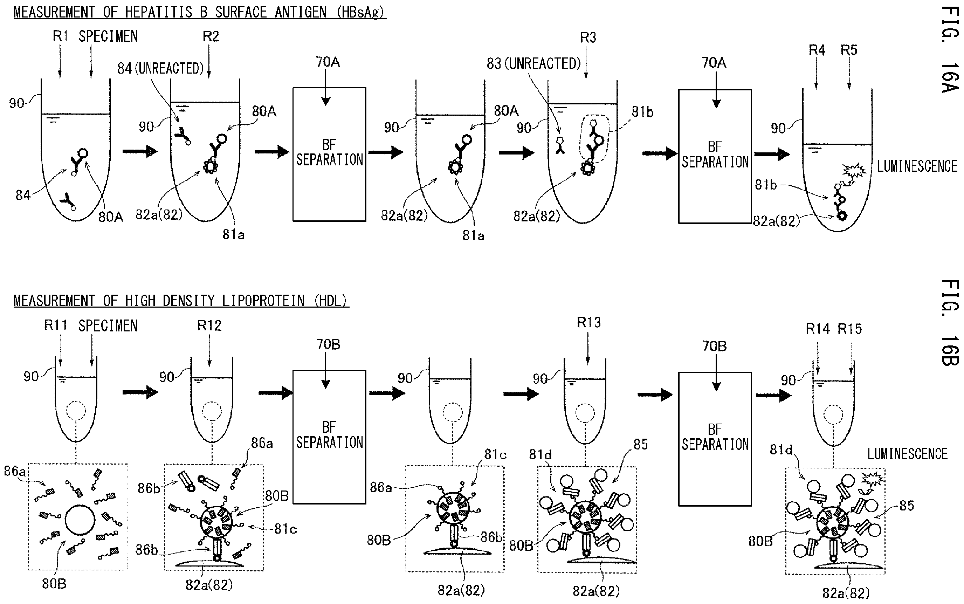

Next, a specific example of measurement performed by the analyzer 100 is described. As shown in FIG. 16, immunoassay is performed by use of the R1 reagent to the R5 reagent. Here, as a measurement example of the target component 80, an example in which the target component 80A is hepatitis B surface antigen (HBsAg) (FIG. 16A), and an example in which the target component 80B is HDL (high density lipoprotein) (FIG. 16B) are described.

### <Measurement of hepatitis B surface antigen (HBsAg)>

First, a specimen containing the target component 80A, and the R1 reagent are dispensed in a reaction container 90. The R1 reagent contains a capture substance 84, and reacts with the target component 80A to be bound thereto. The capture substance 84 includes a binding substance for allowing the capture substance 84 to bind to the carrier 82 (magnetic particle 82a).

For binding between the binding substance and the carrier, a combination such as: biotin and avidins; hapten and anti-hapten antibody; nickel and histidine tag; or glutathione and glutathione-S-transferase, can be used, for example.

For example, the capture substance 84 is an antibody modified with biotin (biotin antibody). That is, the capture substance 84 is an anti-HBs antibody which specifically binds to the target component 80A, and is modified with biotin as the binding substance.

Next, the R2 reagent is dispensed into the reaction container 90. The R2 reagent contains the magnetic particles 82a as the carrier 82. The magnetic particle 82a binds to the binding substance of the capture substance 84. The magnetic particle 82a is a magnetic particle having immobilized thereon streptavidin which binds to biotin (StAvi-bound magnetic particle). Streptavidin of the StAvi-bound magnetic particle reacts with biotin as the binding substance of the capture substance 84, and binds to biotin. As a result, the target component 80A and the capture substance 84 bind to the magnetic particle 82a.

A complex 81a of the target component 80A and the capture substance 84 formed on the magnetic particle 82a is separated from unnecessary components such as unreacted capture substance 84, through a first washing process (primary BF separation process). Through the first washing process, unnecessary components are removed from the reaction container 90. The first washing process is performed by use of the washing process selected by the controller 12. For the first washing process, as the washing liquid 70A to be used, a washing liquid 70A for hepatitis B surface antigen, corresponding to hepatitis B surface antigen as the target component 80A, is selected. The washing liquid 70A for hepatitis B surface antigen contains a surfactant and a salt each at a predetermined concentration or higher, for example.

Next, the R3 reagent is dispensed into the reaction container 90. The R3 reagent contains the labeled substance 83, and reacts with the target component 80A to be bound thereto. As a result, a complex 81b including the target component 80A, the labeled substance 83, and the capture substance 84 is formed on the magnetic particle 82a. In the example shown in FIG. 16A, the labeled substance 83 is an ALP (alkaline phosphatase) labeled antibody. That is, the labeled substance 83 is an anti-HBs antibody which specifically binds to the target component 80A, and is labeled with ALP.

The complex 81b formed on the magnetic particle 82a is separated from unnecessary components such as unreacted labeled substance 83, through a second washing process (secondary BF separation process). Through the second washing process, unnecessary components are removed from the reaction container 90. The second washing process is performed by use of the washing process selected by the controller 12. For the second washing process, as the washing liquid 70A to be used, the washing liquid 70A for hepatitis B surface antigen which is the same as the washing liquid 70A selected for the first washing process is selected.

Then, the R4 reagent and the R5 reagent are dispensed into the reaction container 90. The R4 reagent contains a buffer. The complex 81b bound to the magnetic particle 82a is dispersed in the buffer. The R5 reagent contains a chemiluminescent substrate. The buffer contained in the R4 reagent has a composition that facilitates reaction between the substrate and the label (enzyme) of the labeled substance 83 included in the complex 81b. Light is generated as a result of reaction between the substrate and the label, and the intensity of the generated light is measured by the analysis unit 13.

Thus, the present embodiment includes: a step of performing the first washing process by use of the selected washing process; a step of causing the complex to bind to the labeled substance after the first washing process; a step of performing the second washing process by use of the selected washing process; and a step of measuring a signal based on the labeled substance after the second washing process. Through the first washing process and the second washing process performed in this manner, a higher washing effect can be obtained. Also in this case, since details of an appropriate washing process are selectable, such as use of the selected washing liquid 70A, the measurement can be effectively suppressed from being hindered by influence of the washing process.

### <Measurement of high density lipoprotein (HDL)>

FIG. 16B shows a measurement example in which the target component 80B is HDL (which is lipoprotein) and the function of HDL is evaluated. More specifically, the measurement item to be evaluated is the lipid incorporating function of HDL. HDL is mainly composed of phospholipid, cholesterol, apoprotein, triglyceride, and the like, and has a characteristic of incorporating cholesterol therein. The lipid incorporating function can be evaluated on the basis of the amount of cholesterol incorporated in HDL.

First, a specimen containing the target component 80B, and an R11 reagent are dispensed into a reaction container 90. The R11 reagent contains a capture substance 86a for binding to a labeled substance 85, and binds to the target component 80B.

For binding between the binding substance and the labeled substance 85, a combination such as: biotin and avidins; hapten and anti-hapten antibody; nickel and histidine tag; or glutathione and glutathione-S-transferase, can be used, for example.

For example, the capture substance 86a is cholesterol having DNP (dinitrophenyl group) as a tag added thereto. The cholesterol is incorporated into HDL, which is the target component 80B, to be integrated therein.

Next, an R12 reagent is dispensed into the reaction container 90. The R12 reagent contains the magnetic particles 82a as the carrier 82. In addition, the R12 reagent contains a capture substance 86b for binding to the magnetic particle 82a, and the capture substance 86b reacts with the target component 80B and the magnetic particle 82a, to be bound thereto, respectively. In FIG. 16B, for convenience of drawing, only a part of the surface of the magnetic particle 82a is shown in an enlarged manner.

As the magnetic particles 82a, the magnetic particles in the R2 reagent can be used in common. That is, the magnetic particle 82a is a magnetic particle having immobilized thereon streptavidin which binds to biotin (StAvi-bound magnetic particle). In this case, the capture substance 86b of the R12 reagent is an anti-apoA1 antibody which specifically binds to apoA1, which is apolipoprotein forming HDL. The capture substance 86b is modified with biotin as the binding substance for binding to the magnetic particle 82a. Accordingly, a complex 81c in which the target component 80B having incorporated therein the capture substance 86a is bound to the capture substance 86b is formed on the magnetic particle 82a. In other words, the complex 81c is formed which includes: lipoprotein having incorporated therein the tagged cholesterol (capture substance 86a); and the antibody (capture substance 86b) binding to the lipoprotein.

The complex 81c of the capture substance 86a, the target component 80B, and the capture substance 86b formed on the magnetic particle 82a is separated from unreacted capture substances 86a and 86b, through the first washing process (primary BF separation process). The first washing process is performed by use of the washing process selected by the controller 12. Through the first washing process, unnecessary components such as unreacted capture substances are removed from the reaction container 90. For the first washing process, as the washing liquid 70B to be used, a washing liquid 70B for lipoprotein, corresponding to the lipoprotein as the target component 80B, is selected.

Next, the R13 reagent is dispensed into the reaction container 90. The R13 reagent contains the labeled substance 85 and reacts with the capture substance 86a to be bound thereto. As a result, a complex 81d including the target component 80B, the labeled substance 85, the capture substance 86a, and the capture substance 86b is formed on the magnetic particle 82a. In the example shown in FIG. 16B, the labeled substance 85 is an anti-DNP antibody labeled with ALP.

The complex 81d formed on the magnetic particle 82a is separated from unnecessary components such as unreacted labeled substance 85, through the second washing process. The second washing process is performed by use of the washing process selected by the controller 12. Through the second washing process, unnecessary components such as unreacted labeled substance 85 are removed from the reaction container 90. For the second washing process, as the washing liquid 70B to be used, the washing liquid 70B for lipoprotein which is the same as the washing liquid 70B selected for the first washing process is selected.

Then, the R14 reagent and the R15 reagent are dispensed into the reaction container 90. As the R14 reagent, a buffer similar to that in the R4 reagent can be used. As the R15 reagent, a luminescent substrate similar to that in the R5 reagent can be used. Light is generated as a result of reaction between the substrate and the label, and the intensity of the generated light is measured by the analysis unit 13.

The labeled substance 85 binds to the binding substance modified to the capture substance 86a (cholesterol) having been incorporated in HDL. Thus, the greater the amount of the capture substance 86a incorporated in HDL is, the higher the strength of the signal is. Thus, the strength of the signal serves as an evaluation index for the lipid incorporating function of HDL.

### (Description of measurement process operation)

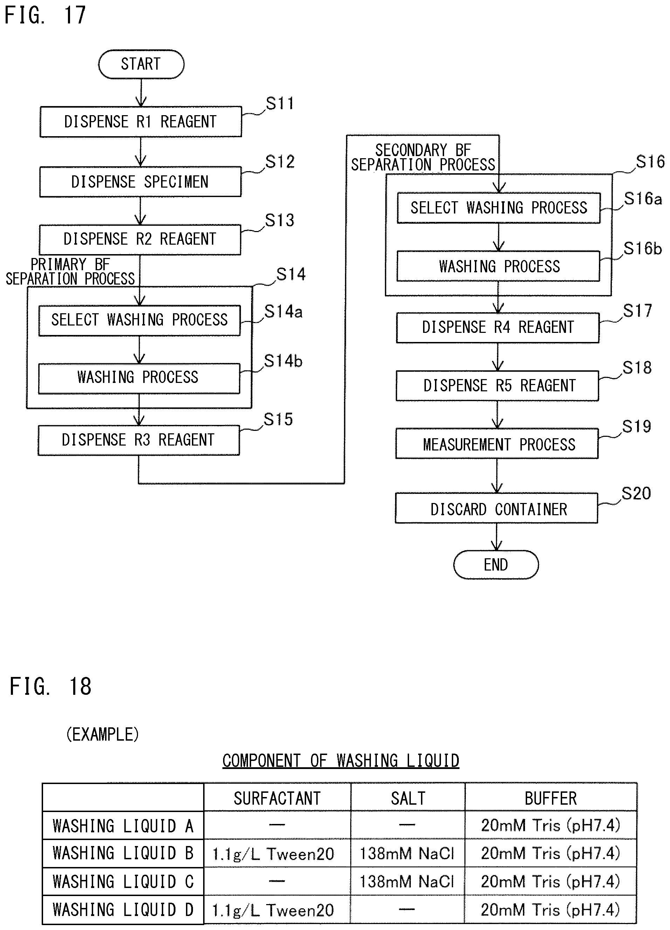

Next, one example of the measurement process operation performed by the analyzer 100 is described with reference to FIG. 17. The following description refers to FIG. 17 with respect to the steps of the measurement process operation, and refers to FIGS. 2A and 2B with respect to the constituents of the analyzer 100. The control of the measurement process operation of the analyzer 100 is performed by the controller 12. Here, an example of the measurement process operation regarding hepatitis B surface antigen (HBsAg) and using the R1 reagent to the R5 reagent is described. However, also regarding high density lipoprotein (HDL), the measurement process operation is the same except that the reagents to be used are changed to the R11 reagent to the R15 reagent.

In step S11, the R1 reagent is dispensed into a reaction container 90. Specifically, a reaction container 90 is supplied from the container supplying unit 34 to the transfer unit 40. The transfer unit 40 transfers the reaction container 90 to the reagent dispensing position 64, and the reagent dispenser 32a dispenses the R1 reagent into the reaction container 90 held in the transfer unit 40. In step S12, the transfer unit 40 transports the reaction container 90 to the specimen dispensing position 63, and the specimen dispenser 31 dispenses a specimen aspirated from a test tube 21a, into the reaction container 90.

After the dispensing, the transfer unit 40 transports the reaction container 90 to the delivering position 61, and the transfer unit 37 takes out the reaction container 90 and sets the reaction container 90 in the reaction unit 10a. In this state, the specimen and the R1 reagent in the reaction container 90 are heated at a predetermined temperature for a predetermined time period.

In step S13, the transfer unit 37 transports the reaction container 90 from the reaction unit 10a to the dispensing port 41a, and the reagent dispenser 32b dispenses the R2 reagent into the reaction container 90. After the dispensing, the transfer unit 37 takes out the reaction container 90 from the dispensing port 41a, and sets the reaction container 90 in the reaction unit 10a. In this state, the specimen, the R1 reagent, and the R2 reagent in the reaction container 90 are heated at a predetermined temperature for a predetermined time period.

In step S14, the primary BF separation process is performed. Specifically, the transfer unit 37 transports the reaction container 90 from the reaction unit 10a to the magnetic collection port 41. At the magnetic collection port 41, the magnetic particles in the reaction container 90 are subjected to pre-magnetic collection by the magnet. Next, the transfer unit 38 transports the reaction container 90 from the magnetic collection port 41 to the washing unit 11.

At this time, in step S14a, the controller 12 selects a washing process, in accordance with the target component 80 contained in the reaction container 90. Thus, details of the washing process such as the kind of the washing liquid 70 and the washing method are determined. In step S14b, the washing unit 11 performs the selected washing process.

In step S15, the transfer unit 38 transports the reaction container 90 from the washing unit 11 to the dispensing port 41b. The reagent dispenser 32c moves to the dispensing port 41b and dispenses the R3 reagent into the reaction container 90. After the dispensing, the transfer unit 38 takes out the reaction container 90 from the dispensing port 41b and sets the reaction container 90 in the reaction unit 10b. In this state, the specimen, the R1 reagent, the R2 reagent, and the R3 reagent in the reaction container are heated at a predetermined temperature for a predetermined time period.

In step S16, the secondary BF separation process is performed. Specifically, the transfer unit 38 transports the reaction container 90 from the reaction unit 10b to the magnetic collection port 41, and subjects the magnetic particles 82a in the reaction container 90 to pre-magnetic collection. Next, the transfer unit 38 transports the reaction container 90 from the magnetic collection port 41 to the washing unit 11.

At this time, in step S16a, the controller 12 selects a washing process that is the same as the primary BF separation process. In step S16b, the washing unit 11 performs the selected washing process. After the washing, the transfer unit 38 transports the reaction container 90 from the washing unit 11 to the relay unit 42.

In steps S17 and S18, the R4 reagent and the R5 reagent are dispensed, respectively. The transfer unit 39 takes out the reaction container 90 from the relay unit 42 and transports the reaction container 90 to the reagent dispenser 32d and the reagent dispenser 32e, sequentially. The reagent dispenser 32d dispenses the R4 reagent into the reaction container 90, and the reagent dispenser 32e dispenses the R5 reagent into the reaction container 90.

After the dispensing, the transfer unit 39 sets the reaction container 90 in the reaction unit 10c. In this state, the specimen, and the R1 reagent to the R5 reagent in the reaction container 90 are heated at a predetermined temperature for a predetermined time period. As a result, the preparation process of a measurement sample is completed.

When the preparation process of the measurement sample is completed, the transfer unit 39 takes out the reaction container 90 from the reaction unit 10c and sets the reaction container 90 in the inter-stage transport unit 36. Then, the raising/lowering device 50 lowers the inter-stage transport unit 36 to transport the reaction container 90 to the second stage 24b (see FIG. 2B).

In step S19, the measurement process of the complex 81 is performed. Specifically, the container transport unit 52 takes out the reaction container 90 from the inter-stage transport unit 36 and transports the reaction container 90 to the analysis unit 13. The analysis unit 13 measures the intensity of light that is generated as a result of reaction between the substrate and the label. The measurement result obtained by the analysis unit 13 is outputted to the controller 12.

After the detection has ended, in step S20, the container transport unit 52 takes out the reaction container 90 having been subjected to the measurement, from the analysis unit 13, and discards the reaction container 90 into the container discard hole 53. In this manner, the measurement process operation of the analyzer 100 is performed.

### [Example] (Example 1)

Next, Example 1 is described. In Example 1, washing processes were performed by use of a plurality of kinds of washing liquids 70 having different compositions, respectively, and influence of each of the washing liquids 70 on the measurement result of the target component 80 was evaluated.

### (1-1) Extraction of HDL fraction

To the serum (0.1 ml) of a patient having dyslipidemia, an equivalent amount of 22% polyethylene glycol 4000 (manufactured by Wako Pure Chemical Industries, Ltd.) was added, and the mixture was suspended. The obtained suspension was left still for 20 minutes at room temperature, and then, was centrifuged at 3000 rpm for 15 minutes at room temperature. Then, the supernatant was collected as an HDL fraction. A fraction denotes an individual component separated from a mixture. The obtained HDL fraction was preserved at 4°C. The obtained abnormal specimen was used as a biological specimen in the following operation.

### (1-2) Formation of complex of HDL and anti-apoAI antibody on carrier

### (i) Preparation of measurement sample

The abnormal specimen was diluted by a reaction buffer to prepare an HDL fraction-containing diluted solution having an ApoAI concentration of 0.2 µg/ml. As the reaction buffer, PBS (phosphate buffered saline) containing 2% BSA (bovine serum albumin) and 2 mM liposome (manufactured by Nippon Fine Chemical Co., Ltd.) was used.

As a control specimen not containing the HDL fraction (ApoAI concentration 0µg/ml), the reaction buffer above was used.

0.5 mM DNP-added cholesterol was added to the reaction buffer so as to have a final concentration of 5 µM, and then, the HDL fraction-containing diluted solution described above was added by a 1/100 amount of the entire amount. The obtained mixture was held at 37°C, and was shaken at 800 rpm for 2 hours to prepare a measurement sample.

### (ii) Formation of complex of HDL having incorporated cholesterol therein and anti-apoAI antibody

30 µl of the prepared measurement sample was taken into another tube, 40 µl of 2.5 ng/µl biotin anti-apoA1 antibody was added thereto, and the mixture was allowed to react at 42°C for 12 minutes. As the biotin anti-apoA1 antibody, an anti-apoA1 antibody (manufactured by SANBIO) that was reduced with 2-Mercaptoethylamine Hydrochloride (manufactured by Nacalai Tesque) and labeled with Biotin-PEAC5-maleimide (manufactured by Dojindo) was used.

Subsequently, 30 µl of HISCL magnetic particles (manufactured by Sysmex Corporation) was added, and the mixture was allowed to react at 42°C for 10 minutes.

Washing operation of: magnetically collecting the magnetic particles; removing the supernatant; and pouring a washing liquid was repeated 3 times, whereby the complex bound to the magnetic particle was washed. Each of the four kinds of washing liquids A to D listed in FIG. 18 was used to perform the washing.

### (1-3) Measurement of amount of cholesterol incorporated in HDL, based on chemiluminescence

### (i) Measurement of amount of DNP-added cholesterol

Magnetic collection was performed to remove the supernatant, 640 ng/µl ALP-labeled anti-DNP antibody was added, and the mixture was allowed to react at 42°C for 10 minutes. As the ALP-labeled anti-DNP antibody, an antibody obtained by removing the Fc region of an anti-DNP antibody (manufactured by Kitayama Labes Co., Ltd.) by pepsin, then, reducing SH with MEA, and then causing the resultant antibody to react with ALP-maleimide was used.

Operation of: magnetically collecting the magnetic particles in the tube; removing the supernatant; and pouring a washing liquid was repeated 3 times, and the complex bound to the magnetic particle was washed. For the washing, the washing liquid, among the washing liquids A to D, that was the same as the washing liquid used in the first-time washing was used.

The magnetic particles in the tube were magnetically collected to remove the supernatant, 50 µl of an HISCL luminescent substrate (manufactured by Sysmex Corporation, the R5 reagent) and 100 µl of the R4 reagent were added, and the mixture was allowed to react at 42°C for 5 minutes. Then, the magnetic particles were magnetically collected, the supernatant was placed on a microplate, and the amount of luminescence was measured by a plate reader.

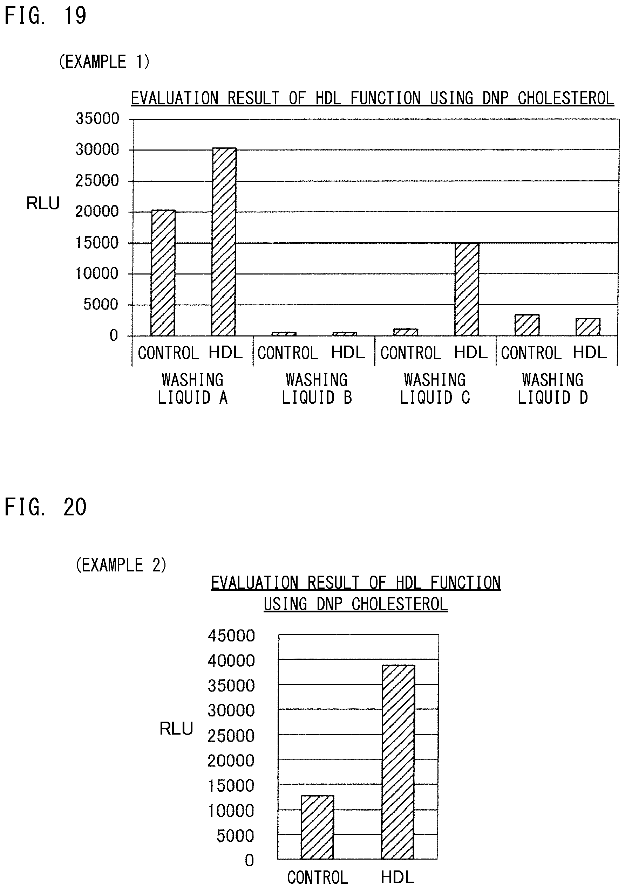

The measurement result is shown in FIG. 19. The vertical axis represents signal strength detected by the plate reader. With reference to FIG. 18 and FIG. 19, in the cases of the washing liquids B and D each containing a surfactant, there was hardly any difference in the signal strength between the measurement sample and the control specimen, whereas in the cases of the washing liquids A and C each not containing a surfactant, there was a clear difference in the signal strength when compared with the signal strength of the control specimen not containing HDL. The washing liquid B has a composition that is used in general in measurement of hepatitis B surface antigen (HBsAg) described above. Thus, it has been confirmed that, depending on the combination of the target component and the washing liquid, there are cases where measurement is hindered by the washing liquid and where measurement is not hindered by the washing liquid.

In the case of the washing liquid C containing a salt (NaCl), the strength of the background signal was reduced when compared with the washing liquid D not containing any salt, and the signal/background ratio was improved by about 25 times when compared with the washing liquid A.

From the above, it has been confirmed that, with respect to the measurement item for evaluating the amount of cholesterol incorporated in HDL (lipid incorporating function of HDL), if the washing process is performed by use of the washing liquid A or C in accordance with the target component, the cholesterol incorporating ability of HDL can be evaluated.

With respect to the washing liquid C, the signal/background ratio is improved as a result of reduction of the background signal, and thus, it has been confirmed that the measurement accuracy can be improved by selecting an appropriate washing liquid.

With respect to the washing liquids B and D, since HDL contains lipid as a major component, the structure of HDL is changed due to influence of the surfactant. As a result, it is considered that DNP-added cholesterol incorporated in HDL is taken out from HDL into the washing liquid. Thus, with respect to a target component 80 that contains lipid, it is appropriate to select a washing liquid that does not contain any surfactant or that contains a surfactant at a concentration lower than a predetermined concentration that would not cause influence on the measurement.

The surfactant functions as a washing component for washing off unnecessary components non-specifically adsorbed, from the complex on the carrier or from the reaction container. Thus, in the case of a washing liquid not containing any surfactant or containing a surfactant at not higher than a predetermined concentration, the background signal could increase. With respect to the washing liquid C containing a salt, the background signal was reduced when compared with the washing liquid A not containing any salt. Thus, for the target component 80 that contains lipid, it is considered to be appropriate to select a washing liquid in which a salt functions as a washing component similarly to a surfactant, and that contains the salt at an appropriate concentration.

### (Example 2)

In Example 2, the washing process is performed by use of the washing liquid C in the analyzer 100 shown in FIGS. 2A and 2B, and measurement operation for evaluating the lipid incorporating function of HDL as the target component 80 was performed.

### (1) Preparation of R11 reagent

To PBS that contained 2% BSA and 2 mM liposome (manufactured by Nippon Fine Chemical Co., Ltd.), 0.5 mM DNP-added cholesterol was added so as to have a final concentration of 5 µM, and the resultant mixture was used as the R11 reagent.

### (2) Preparation of R12 reagent

The HISCL magnetic particles and 2.5 ng/µl biotin anti-apoA1 antibody were mixed at a mixing ratio of 3:4, and the antibody was allowed to bind to the magnetic particles under a condition of 25°C, 2 hours, and 1200 rpm. After the magnetic particles were magnetically collected, the supernatant was removed, washing was performed with 2%BSA, and then, the mixture was suspended in 2%BSA. At this time, the R12 reagent was prepared such that the concentration of the magnetic particles became twice of the initial concentration thereof.

### (3) Preparation of R13 reagent

An ALP-labeled anti-DNP antibody was diluted in Starting block (TBS) (manufactured by Thermo Scientific), whereby a 640 ng/µl detection antibody solution was prepared as the R13 reagent. As the ALP-labeled anti-DNP antibody, an antibody obtained by removing the Fc region of an anti-DNP antibody (manufactured by Kitayama Labes Co., Ltd.) by pepsin, then, reducing SH (thiol group) with MEA (mercaptoethylamine), and then, causing the resultant antibody to react with ALP-maleimide was used.

### (4) Preparation of measurement sample

The abnormal specimen was diluted with 2.25% BSA/PBS, and an oxidizing agent (8.8 M hydrogen peroxide, 1.76 mM sodium nitrite, and 0.86 mM diethylenetriamine pentaacetic acid (DTPA)) was added. At this time, the addition was performed such that the final concentrations of ApoA1 and BSA became 10 µg/ml and 2%, respectively. The mixture was shaken under a condition of 37°C, 1 hour, and 800 rpm, and was diluted with 2% BSA/PBS such that the concentration of ApoA1 became 1 µg/ml.

The R11 reagent, the R12 reagent, the R13 reagent, and the measurement sample that were prepared, and an HISCL R4 reagent and an HISCL R5 reagent that were commercially available were disposed in the analyzer 100, and the following steps were performed.

### (5) Evaluation of cholesterol incorporating function

90 µl of the R1 reagent was dispensed into an HISCL cuvette, 10 µl of the measurement sample was added thereto, and the mixture was agitated and then left still in the reaction unit 10 at 42°C for 12 minutes. Subsequently, 30 µl of the R2 reagent was dispensed, and the mixture was agitated and then left still in the reaction unit 10 for the same time period. The washing liquid C (138 mM NaCl, 20 mM Tris buffer (pH7.4)) was selected, the primary BF separation process was performed, and the complex 81 on the magnetic particle 82a was washed 3 times. Then, 100 µl of the R3 reagent was dispensed, and the mixture was agitated and then left still in the reaction unit 10 at 42°C for 10 minutes. The washing liquid C was selected, the secondary BF separation process was performed, and the complex was washed 3 times. Then, the R4 reagent and the R5 reagent were dispensed by 50 µl and 100 µl, respectively, and the mixture was agitated and then left still in the reaction unit 10 at 42°C for 5 minutes. The chemiluminescence signal was detected by the analysis unit 13.

The detection result is shown in FIG. 20. The control specimen is the same as that in Example 1 described above. Also in FIG. 20, by selecting the washing liquid C to be used in the washing process for the complex 81, a clear difference in the signal strength was observed when compared with the signal strength of the control specimen corresponding to the background. Thus, it has been confirmed that the cholesterol incorporating ability of HDL can be evaluated by the analyzer 100.

### [Modification]

It should be noted that the embodiment disclosed herein is merely illustrative in all aspects and should not be considered as restrictive. The scope of the present disclosure is not defined by the description of the above embodiment but by the scope of the claims, and includes meaning equivalent to the scope of the claims and all changes (modifications) within the scope of the claims.

For example, as a measurement method that is different from the measurement method shown in the embodiment described above, the measurement may be performed by use of an immune complex transfer method. In the immune complex transfer method, the complex 81 formed on the carrier 82 is caused to be released from the carrier 82, and the released complex 81 is extracted. For example, a releasing reagent is dispensed into a reaction container 90 holding a sample containing the complex 81 formed on the carrier 82. The carrier 82 is captured in the reaction container 90, and the supernatant in the container is aspirated to be transferred into another reaction container 90, whereby the free complex 81 present in the supernatant is extracted. Then, if the complex 81 transferred into the reaction container 90 is caused to bind to another carrier 82, the measurement can be performed in the procedure of step S15 and thereafter shown in FIG. 17. With the immune complex transfer method, unnecessary components non-specifically adsorbed to the carrier 82 can be separately removed from the complex 81, and thus, further accurate measurement with reduced background signal can be realized.

### DESCRIPTION OF THE REFERENCE CHARACTERS

10: reaction unit
11: washing unit
12: controller
13: analysis unit
14: setting unit
23: washing liquid storage part
23a: first storage part
23b: second storage part
35: reagent storage part
44: washing port
55: input unit
56: supply channel
70: washing liquid
80: target component
81: complex
82: carrier
82a: magnetic particle
83: labeled substance
91: measurement order
100: analyzer

## Claims

1. An analysis method comprising:
a component to be analyzed being set;
bringing a carrier and a complex that includes the component into contact with each other, to form the complex on the carrier;
selecting a washing process in accordance with the component; and
analyzing the component, with use of the washing process selected in accordance with the set component.

2. The analysis method of claim 1, wherein
when lipoprotein is set as the component,
the analysis method includes forming the complex that includes: lipoprotein having tagged cholesterol incorporated therein; and an antibody that binds to the lipoprotein.

3. The analysis method of claim 1 or 2, wherein
a washing liquid to be used in the washing process is selected from a plurality of kinds of washing liquids in which at least washing liquid components contained therein or proportions of the washing liquid components are different.

4. The analysis method of claim 3, wherein
a washing liquid that does not contain any surfactant or that contains a surfactant at a concentration of less than 1.1 g/L is selected in accordance with the component.

5. The analysis method of claim 3 or 4, wherein
a washing liquid that contains a predetermined washing liquid component is selected in accordance with the component.

6. The analysis method of claim 5, wherein
the predetermined washing liquid component is a salt.

7. The analysis method of any one of claims 3 to 6, wherein
the washing liquid is a washing liquid for a liquid distribution channel.

8. The analysis method of any one of claims 3 to 7, comprising
preparing a washing liquid to be used in the washing process, by mixing one washing liquid and another washing liquid from among a plurality of washing liquids.

9. The analysis method of any one of claims 1 to 8, wherein
the component to be analyzed being set includes:
obtaining information that specifies the component; and
selecting the washing process on the basis of the obtained information that specifies the component.

10. The analysis method of claim 9, wherein
the information that specifies the component is a measurement order in which a measurement item is set for each specimen, and
the analysis method comprises
obtaining the measurement order for the specimen, and selecting the washing process in accordance with the measurement item in the measurement order.

11. The analysis method of any one of claims 1 to 10, wherein
the washing process includes:
magnetically collecting the complex;
supplying a washing liquid to be used in the washing process; and
removing the washing liquid.

12. The analysis method of any one of claims 1 to 11, wherein
analysis of the component is performed through immunoassay using antigen-antibody reaction.

13. The analysis method of any one of claims 1 to 12, wherein
the carrier is a solid-phase particle.

14. The analysis method of claim 13, wherein
the solid-phase particle is a magnetic particle.

15. An analyzer comprising:
a setting unit configured to set a component to be analyzed;
a reaction unit configured to bring a carrier and a complex that includes the component into contact with each other, to form the complex on the carrier;
a washing unit configured to perform a washing process selected in accordance with the set component, the washing process being for washing the complex formed in the reaction unit;
an analysis unit configured to analyze the component after the washing process has been performed by the washing unit; and
a controller configured to select the washing process in accordance with the component.
